# EUROPÄISCHE PATENTSCHRIFT

(11) **EP 1 903 105 B1**
(45) Veröffentlichungstag und Bekanntmachung des Hinweises auf die Patenterteilung: **21.04.2010**
(21) Anmeldenummer: 06121090.2
(22) Anmeldetag: 22.09.2006
(51) Int. Cl.: C12N 1/20

(54) **Verfahren zur fermentativen Herstellung von Proteinen**
Process for the production of proteins by fermentation
Procédé pour la production de protéines par fermentation

(43) Veröffentlichungstag der Anmeldung: 26.03.2008
(73) Patentinhaber: Wacker Chemie AG, 81737 München (DE)
(72) Erfinder: Dassler, Tobias, 81825 München (DE); Wich, Günter, 81377 München (DE); Schmid, Gerhard, 82121 Gauting (DE)
(74) Vertreter: Potten, Holger

(56) Entgegenhaltungen:
- EP-A2- 0 338 410
- EP-A2- 0 448 093
- US-A- 5 223 482
- KANAMORI T ET AL: "Expression and excretion of human pancreatic secretory trypsin inhibitor in lipoprotein-deletion mutant of Escherichia coli." GENE. 30 JUN 1988, Bd. 66, Nr. 2, 30. Juni 1988 (1988-06-30), Seiten 295-300, XP002411679 ISSN: 0378-1119
- MORISHITA HIDEAKI ET AL: "Novel factor Xa and plasma kallikrein inhibitory-activities of the second Kunitz-type inhibitory domain of urinary trypsin inhibitor" THROMBOSIS RESEARCH, Bd. 73, Nr. 3-4, 1994, Seiten 193-204, XP002411680 ISSN: 0049-3848
- ZHANG W Y ET AL: "Alterations of the carboxyl-terminal amino acid residues of Escherichia coli lipoprotein affect the formation of murein-bound lipoprotein." THE JOURNAL OF BIOLOGICAL CHEMISTRY. 25 SEP 1992, Bd. 267, Nr. 27, 25. September 1992 (1992-09-25), Seiten 19560-19564, XP002411681 ISSN: 0021-9258
- KENZO NAKAMURA ET AL: "DNA SEQUENCE OF THE GENE FOR THE OUTER MEMBRANE LIPOPROTEIN OF E. COLI: AN EXTREMELY AT-RICH PROMOTER" CELL, MIT PRESS, CAMBRIDGE, MA,, US, Bd. 18, Nr. 4, 1. Dezember 1979 (1979-12-01), Seiten 1109-1117, XP000603528 ISSN: 0092-8674
- GAUCHERON FRÉDÉRIC: "The minerals of milk." REPRODUCTION, NUTRITION, DEVELOPMENT. 2005 JUL-AUG, Bd. 45, Nr. 4, Juli 2005 (2005-07), Seiten 473-483, XP002411682 ISSN: 0926-5287
- "Typical Analysis of Bacto(TM) Tryptone" Gefunden im Internet: URL:http://www.bd.com/ds/technicalCenter/t ypicalAnalysis/typ-tryptone.pdf> [gefunden am 2007-12-20]
- MILLER K.W. ET AL.: APPL ENVIRON MICROBIOL, Bd. 64, 1998, Seiten 14-20,

## Beschreibung

Die Erfindung betrifft ein Verfahren zur fermentativen Herstellung von heterologen Proteinen unter Verwendung eines Escherichia coli Stammes mit einer Lipoprotein-Mutation.

Der Markt für rekombinante Proteinpharmazeutika (Pharmaproteine/Biologics) ist in den letzten Jahren stark gewachsen. Besonders wichtige Proteinpharmazeutika sind dabei eukaryontische Proteine, vor allem Säugetierproteine und menschliche Proteine. Beispiele wichtiger Pharmaproteine sind Cytokine, Wachstumsfaktoren, Proteinkinase, Protein- und Peptidhormone sowie Antikörper und Antikörperfragmente. Aufgrund der immer noch sehr hohen Produktionskosten für Pharmaproteine wird laufend nach effizienteren und damit kostengünstigeren Verfahren und Systemen für deren Herstellung gesucht.

Generell werden rekombinante Proteine entweder in Säugerzellkulturen oder in mikrobiellen Systemen hergestellt. Mikrobielle Systeme weisen gegenüber Säugerzellkulturen den Vorteil auf, dass auf diese Weise rekombinante Proteine in kürzerer Zeit und zu geringeren Kosten hergestellt werden können. Für die Produktion von rekombinanten Proteinen eignen sich daher vor allem Bakterien. Aufgrund seiner sehr gut untersuchten Genetik und Physiologie, der kurzen Generationszeit und der einfachen Handhabung ist das Gram-negative Enterobakterium Escherichia coli gegenwärtig der am häufigsten verwendete Organismus zur Produktion rekombinanter Proteine. In E. coli können rekombinante Proteine üblicherweise auf verschiedene Arten hergestellt werden:
1. intrazelluläre Produktion als lösliches Protein;
2. intrazelluläre Produktion als Einschlusskörperchen ("inclusion bodies");
3. Sekretion in das Periplasma.

Die Anhäufung des Zielproteins im Periplasma hat gegenüber der intrazellulären Produktion verschiedene Vorteile: 1) der N-terminale Aminosäurerest des sekretierten Zielproteins muss nicht zwangsläufig Methionin sein, sondern kann identisch zu der natürlichen Startaminosäure des Produkts sein, 2) ist die Protease-Aktivität im Periplasma bzw. Fermentationsmedium deutlich geringer als im Cytoplasma, 3) wird die Ausbildung von evtl. notwendigen Disulfidbrücken unter den oxidativen Bedingungen und durch die Chaperone des Periplasmas ermöglicht.

E. coli hat verschiedene Systeme, um Proteine durch die Cytoplasmamembran ins Periplasma zu transportieren. Für die sekretorische Herstellung rekombinanter Proteine wird am häufigsten das Sec-System verwendet. Dabei wird das Gen des gewünschten Proteins mit einer Signalsequenz von solchen Proteinen funktionell verknüpft, die normalerweise von E. coli mit Hilfe des Sec-Apparates exportiert werden (z. B. PhoA, OmpA, OmpF, StII, Lpp, MalE). Es können aber auch heterologe Signalsequenzen, wie z. B. eine α-CGTase-Signalsequenz, verwendet werden, die ebenfalls vom Sec-Apparat von E. coli erkannt werden (EP0448093). Im Falle des Sec-Systems werden die Proteine im ungefalteten Zustand durch die Cytoplasma-Membran transportiert und erst anschließend im Periplasma gefaltet.

Der Produktionsprozess des rekombinanten Proteins gliedert sich dabei immer in zwei Teile. Der erste Teil ist die Fermentation, welche zum Rohprodukt führt. Als Rohprodukt wird in diesem Fall das Fermentationsergebnis bezeichnet, welches das rekombinante Protein und außerdem noch verunreinigende wirtseigene Proteine enthält. Der zweite Teil des Produktionsprozesses umfasst die Aufreinigung des rekombinanten Proteins ausgehend vom Rohprodukt.

Aufwand und Kosten der Herstellung des rekombinanten Proteins werden neben den Produktionskosten des Rohproduktes, welches direkt nach der Fermentation als Gemisch umfassend das rekombinante Protein sowie Wirtsproteine vorliegt, auch wesentlich bestimmt durch die Aufreinigungskosten des Rohproduktes zum erwünschten rekombinanten Protein. Die Aufreinigung erfolgt in den meisten Fällen über mehrere Stufen mittels chromatographischer Verfahren. Dabei spielt die Abreinigung von verunreinigenden Wirtsproteinen, die z. T. immunogen oder toxisch sind, eine bedeutende Rolle. Hierbei weisen sowohl die intrazelluläre Produktion, wie die periplasmatische Produktion folgende Nachteile auf:
1. Die Zellen müssen aufgeschlossen werden.
2. Die Zielproteine müssen aus einer Vielzahl von Wirtsproteinen aufgereinigt werden.
Bei intrazellulärer Produktion kommt noch dazu, dass die Zielproteine häufig in teilweise fehlerhafter Faltung vorliegen.

Besonders bevorzugt sind daher Produktionsverfahren für rekombinante Proteine in E. coli, bei denen das Zielprotein in hoher Ausbeute und in der korrekten Faltung direkt ins Fermentationsmedium sekretiert wird.

In der Literatur sind eine Reihe von E. coli Stämmen und Prozesse mit E. coli Stämmen offenbart, durch die eine Sekretion von rekombinanten Proteinen ins Fermentationsmedium erreicht wird (Review s. Shokri et al., Appl. Microbiol. Biotechnol. 60 (2003), 654 - 664; Mergulhao et al., Biotechnology Advances 23 (2005), 177 - 202, Choi and Lee, Appl. Microbiol. Biotechnol. 64 (2004), 625 - 635).

In EP 0338410 und EP 0448093 ist die Herstellung und die Verwendung einer "Sekretormutante" von E. coli offenbart, die eine massive Protein-Sekretion in das Fermentationsmedium aufweist. Als Ausgangsstamm zur Herstellung von geeigneten E. coli-Sekretormutanten kann man insbesondere Zellen mit einer minA- und/oder minB-Mutation (z. B. DS410) oder Zellen, die in einem Protein oder in mehreren Proteinen der äußeren Membran mutiert sind (z. B. BW7261), verwenden. Diese Zellen wurden zusätzlich einer Mutagenese-Prozedur, z. B. durch Behandlung mit N-Methyl-N'-nitro-N-nitrosoguanidin, unterzogen. Anschließend erfolgt dann z. B. eine Selektion auf Resistenz gegen die Zellwand-aktive Substanz D-Cycloserin, oder es folgt ein Screening auf verbesserte Protein-Sekretion durch Analyse der Hofbildung in einem Amylopektin-Azur-Agarmedium, wenn man das sekretierbare, stärkeabbauende Enzym α-Cyclodextrin-Glycosyl-Transferase (αCGTase) als Indikatorprotein verwendet. Mit einer auf diese Weise erzeugten Sekretormutante konnten heterologe Proteine, wie z. B. eine αCGTase bzw. ein Hirudin-Derivat mit extrazellulären Ausbeuten von 240 mg/l bzw. von 2,63 g/l hergestellt werden. Ein großer Nachteil dieser Sekretormutanten liegt in der aufwändigen Herstellungsprozedur mittels Mutagenese und Screening. Außerdem umfasst die Herstellung einen ungerichteten Mutageneseschritt, der zusätzlich zur gewünschten Mutation zu unerwünschten Mutationen führen kann.

EP 0497757 beschreibt die Herstellung von E. coli Stämmen, die biologisch aktive, also korrekt gefaltete heterologe Proteine in das Anzuchtmedium sekretieren, dadurch gekennzeichnet, dass E. coli Stämme mit mutagenen Agenzien behandelt werden, über Resistenz gegen den Bakteriophagen T7 nach Mutanten gesucht wird, die Veränderungen in der äußeren Membran haben und diese auf die Eigenschaft "Proteinsekretion in das Medium" getestet werden. Die Proteinausbeuten, die im Medium mit solchen Stämmen erreicht werden, sind jedoch sehr niedrig (< 5 mg/l). Auch hier liegt ein weiterer Nachteil in der aufwändigen und schlecht reproduzierbaren Herstellung solcher Stämme.

Die im Stand der Technik beschriebenen Ansätze weisen mindestens einen der folgenden Nachteile auf:
a) entweder können mit einem Produktionssystem nur homologe oder ganz spezielle Proteine extrazellulär in ausreichend hoher Ausbeute hergestellt werden, oder
b) wenn ein System prinzipiell für die Herstellung verschiedenartiger Proteine geeignet ist, wurden damit bisher aus wirtschaftlicher Sicht nur geringe Ausbeuten erzielt, oder
c) es müssen im Anschluss an die Kultivierung noch weitere Schritte, wie z. B. die Abspaltung des Zielproteins von einem Fusionspartner, folgen, was die Aufarbeitung aufwändiger macht, oder
d) die Erzeugung eines Sekretionsstammes, der in der Lage ist, Proteine mit hoher Ausbeute in das Fermentationsmedium zu sekretieren, ist nur durch ein aufwändiges Mutagenese- und Screening-Verfahren möglich.

Daneben besteht prinzipiell die Möglichkeit der Verwendung von so genannten "leaky"-Stämmen. Darunter sind Mutanten von E. coli oder Salmonella zu verstehen, die einen Defekt in der äußeren Membran aufweisen und deshalb periplasmatische Proteine teilweise in das Fermentationsmedium entlassen. Es handelt sich dabei um einen unspezifischen Mechanismus (Lazzaroni und Portalier, 1981, J. Bact. 145, 1351 - 58). Ein Beispiel für solche "leaky"-Mutanten sind Stämme mit veränderten Lipoprotein-Anteilen in der äußeren Membran (z. B. lpp-Mutanten) (Hirota et al., 1977, Proc. Natl. Acad. Sci. USA 74, 1417 - 20; Yem und Wu, 1978, J. Bact. 133, 1419 - 26; Suzuki et al., 1978, Mol. Gen. Genet. 167, 1 - 9).

Es ist bekannt, dass lpp-Mutanten zelleigene periplasmatische Proteine, wie z. B. die alkalische Phosphatase PhoA oder die RNase I, in das Fermentationsmedium entlassen. Solche Stämme sind extrem empfindlich gegenüber EDTA, verschiedenen Detergenzien und Farbstoffen (Fung et al., 1978, J. Bact. 133, 1467 - 71; Suzuki et al., 1978, Mol. Gen. Genet. 167, 1 - 9; Hirota et al., 1977, Proc. Natl. Acad. Sci. USA 74, 1417 - 20).

Der Einsatz von lpp-Mutanten von E. coli zur Produktion von heterologen Proteinen in technischem Maßstab wurde bisher nicht beschrieben. Es wird hingegen immer darauf hingewiesen, dass "leaky"-Stämme von E. coli, zu denen auch die lpp-Mutanten zählen, nicht ausreichend robust und somit für eine großtechnische Kultivierung nicht geeignet sind (EP0357391; Wan und Baneyx, 1998, Protein Expres. Purif. 14, 13 - 22; Shokri et al., 2003, Appl. Microbiol. Biotechnol. 60: 654 - 64; Ray et al., 2002, Protein Expres. Purif. 26, 249 - 59).

Nur in wenigen Publikationen geht die Verwendung von lpp-Mutanten im Labormaßstab über die reine Charakterisierung dieser Stämme hinaus. So wurde eine lpp-Deletionsmutante von E. coli aufgrund ihrer Eigenschaft, periplasmatische Proteine teilweise in das Fermentationsmedium zu entlassen, als Werkzeug eingesetzt, um potentielle Virulenz-Gene aus pathogenen Mikroorganismen, die für exportierte Proteine mit abspaltbaren Signalpeptiden kodieren, über ein Hofbildungs-Screening zu identifizieren (Giladi et al., 1993, J. Bact. 175, 4129 - 36). Die heterologen Zielproteine wurden dabei als Fusionsproteine mit der E. coli eigenen periplasmatischen Alkalischen Phosphatase erzeugt und als Fusionsproteine in das Fermentationsmedium sekretiert.

In einem anderen Ansatz wurde die extrazelluläre Anhäufung eines Fusionsproteins bestehend aus dem Maltose-Bindeprotein von E. coli (MBP) und dem Bacteriocin Pediocin AcH (PapA) aus Pediococcus acidilactici unter Verwendung einer lpp-Insertionsmutante von E. coli, beschrieben (Miller et al., 1998, Appl. Environ. Microbiol. 64, 14 - 20). Auch hier wurde das heterologe Zielprotein als Fusionsprotein mit einem zelleigenen Protein ins Fermentationsmedium sekretiert.

Beide Veröffentlichungen beschreiben die Produktion der Fusionsproteine in Schüttelkolben im Labormaßstab in komplexen und teuren Labormedien (z. B. Luria-Bertani Broth).

Darüber hinaus ist in Kanamori et al. (1988, Gene 66, 295 - 300), Morishiva et al. (1994, Thrombosis Research 73, 193 - 204) und US5223482 die Verwendung der lpp-Mutante JE5505 (Suzuki et al., 1978, Mol. Gen. Genet. 167, 1 - 9) zur extrazellulären Herstellung von eukaryontischen Polypeptiden, die aus maximal 70 Aminosäuren und damit vergleichsweise einfach aufgebaut sind, offenbart. Das jeweils für die Kultivierung verwendete Minimalsalzmedium M9CA enthält zudem mit den supplementierten Casaminosäuren eine teure Komplexkomponente. In einem Fermentationsprozess konnten nur geringe, für einen wirtschaftlichen Prozess uninteressante extrazelluläre Produkt-Ausbeuten von maximal 50 mg/l erzielt werden, was wahrscheinlich auf die mangelnde Robustheit des Stammes unter diesen Fermentationsbedingungen zurückzuführen ist.

Es ist ferner beschrieben, dass lpp-Mutanten Vesikel der äußeren Membran abschnüren (McBroom und Kuehn, 12.5.2005. Kapitel 2.2.4, Outer Membrane Vesicles. In A. Böck, R. Curtiss III, J. B. Kaper, F. C. Neidhardt, T. Nyström, K. E. Rudd, and C. L. Squires (ed.), EcoSal-Escherichia coli and Salmonella: cellular and molecular biology. [Online.] http://www.ecosal.org. ASM Press, Washington, D.C.) und dass die in das Fermentationsmedium abgegebenen Proteine in diesen Vesikeln enthalten sind (Kesty und Kuehn, 2004, J. Biol. Chem. 279, 2069 - 76). Dadurch werden die Proteine dem üblichen Prozess der Disulfid-Brückenbildung und den periplasmatischen Isomerisierungssystemen, die für die Faltung komplexer Proteine im Periplasma notwendig sind, entzogen. Der Durchschnittsfachmann geht deshalb davon aus, dass dies bei komplexen heterologen Proteinen zu fehlerhafter oder unvollständiger Faltung führt.

Sowohl eine Produktion von Proteinen als Fusionsproteine, als auch eine Produktion nicht korrekt gefalteter Proteine ist unerwünscht, da dadurch aufwändige und teure Nachbehandlungen des Zielproteins notwendig sind. So muss bei Fusionsproteinen das gewünschte Zielprotein aufwändig durch spezifische Chemikalien oder Enzyme von dem Fusionspartner abgespalten und gereinigt werden. Im Falle von fehlgefalteten Proteinen sind schwierige Denaturierungs- und Rückfaltungsprozeduren notwendig.

Aufgabe der vorliegenden Erfindung ist es, ein Verfahren zur Produktion eines heterologen Proteins in technischem Maßstab mittels eines E. coli-Stammes in einem Fermentationsmedium bereitzustellen, bei welchem das Protein in hoher Ausbeute in das Fermentationsmedium sekretiert wird, und das heterologe Protein ohne weitere Nachbehandlung direkt aus dem Fermentationsmedium aufgereinigt werden kann.

Diese Aufgabe wird gelöst durch ein Verfahren, gemäß Anspruch 1.

Unter hohen Ausbeuten sind vorzugsweise Protein-Konzentrationen im Fermentationsmedium von vorzugsweise größer 500 mg/l am Ende der Kultivierung zu verstehen, bzw. bei solchen Proteinen, die bereits mit guter Ausbeute produziert werden können, Ausbeuten von mehr als 110 % dessen, was nach dem gegenwärtigen Stand der Technik produziert werden kann.

E. coli-Stämme, welche eine Mutation im lpp-Gen aufweisen, sind in der Literatur beschrieben (Hirota et al., 1977, Proc. Natl. Acad. Sci. USA 74, 1417 - 20; Yem und Wu, 1978, J. Bact. 133, 1419 - 26).

Darüber hinaus sind dem Fachmann Methoden zur Erzeugung von lpp-Mutanten von beliebigen E. coli-Stämmen bekannt. Solche in ihrer Basensequenz von der Sequenz des lpp-Wildtypgens aufgrund von Mutationen abweichenden DNS-Sequenzen werden auch als lpp-Allele bezeichnet. In der Literatur synonym gebrauchte Bezeichnungen für das lpp-Gen sind mlpA oder lpo.

So können lpp-Allele z. B. über eine Transduktion mittels P1-Phagen oder Konjugation aus einem Stamm mit lpp-Mutation auf einen lpp-Wildtypstamm übertragen werden, wobei das lpp-Wildtypgen gegen das lpp-Allel ersetzt wird.

Darüber hinaus sind dem Fachmann weitere Methoden zur Erzeugung von lpp-Allelen bekannt. Derartige lpp-Allele werden in der Regel der Einfachheit halber zunächst in vitro erzeugt und anschließend in das Chromosom der Zelle eingebracht, wodurch das ursprünglich vorhandene lpp-Wildtypgen ersetzt und somit eine lpp-Mutante erzeugt wird. Allele des lpp-Gens kann man beispielsweise durch unspezifische oder gezielte Mutagenese mit der DNS des lpp-Wildtypgens als Ausgangsmaterial herstellen. Unspezifische Mutationen innerhalb des lpp-Gens oder des Promotorbereichs des lpp-Gens können z. B. durch chemische Agentien wie Nitrosoguanidin, Ethylmethansulfonsäure u.ä. und/oder durch physikalische Methoden und/oder durch unter bestimmten Bedingungen durchgeführte PCR-Reaktionen erzeugt werden. Methoden zur Einführung von Mutationen an spezifischen Positionen innerhalb eines DNS-Fragmentes sind bekannt. So kann beispielsweise der Austausch einer oder mehrerer Basen in einem DNS-Fragment, das das lpp-Gen und dessen Promotorregion umfasst, mittels PCR unter Verwendung geeigneter Oligonukleotide als Primer erfolgen.

Die mittels der beschriebenen Methoden in vitro erzeugten lpp-Allele können mittels einfacher Standardmethoden in das Chromosom einer Wirtszelle anstelle des lpp-Wildtypgens/Promotors eingebracht werden. Dies kann beispielsweise mittels des in Link et al. (1997, J. Bacteriol. 179: 6228 - 37) beschriebenen Verfahrens zur Einführung chromosomaler Mutationen in ein Gen über den Mechanismus der homologen Rekombination erfolgen. Die Einführung einer chromosomalen Deletion des gesamten lpp-Gens oder eines Teils davon ist beispielsweise mit Hilfe des λ-Red Rekombinase-Systems nach der von Datsenko und Wanner (2000, Proc. Natl. Acad. Sci. U S A. 97: 6640 - 5) beschriebenen Methode möglich.

Die DNS-Sequenz des lpp-Gens von E. coli (SEQ ID No. 1) kodiert für ein Lpp-Protein mit der Sequenz SEQ ID No. 2. Die ersten 60 Nukleotide kodieren dabei für das Signalpeptid, welches die Sekretion des Lpp-Proteins in das Periplasma steuert und das während dieses Translokationsvorgangs wieder abgespalten wird. Die Promotorregion des lpp-Gens ist in Inouye und Inouye (1985, Nucleic Acids Res. 13, 3101 - 10) definiert.

Der Nachteil der Lpp-Proteinmenge im Vergleich zu Zellen des Wildtyp-Stammes W3110 (ATTC: 27325) kann z. B. durch eine immunologische Quantifizierung des Lpp-Proteins mit Hilfe von Anti-Pal-Antikörpern geschehen (Cascales et al., 2002, J. Bacteriol. 184, 754 - 9).

Bei den in lpp-Mutanten herstellbaren Proteinen handelt es sich um heterologe Proteine. Unter heterologen Proteinen sind Proteine zu verstehen, die nicht zum Proteom, d.h. der gesamten natürlichen Protein-Ausstattung, eines E. coli K12-Stammes gehören. Alle natürlicherweise in E. coli K12-Stämmen vorkommenden Proteine lassen sich aus der bekannten Genomsequenz von E. coli K12 ableiten (Genbank Accession No. NC_000913). Ferner umfasst der Begriff "heterologes Protein" im Sinne der vorliegenden Erfindung keine Fusionsproteine mit einem E. coli Protein.

Die heterologen Proteine weisen dabei mehr als 50 %, bevorzugt mehr als 70 %, besonders bevorzugt mehr als 90 % der spezifischen Aktivität bzw. ihrer Wirkung (Funktion) auf, die charakteristisch für das jeweilige heterologe Protein ist.

Bevorzugt sind heterologe, besonders bevorzugt eukaryontische Proteine, die eine oder mehrere Disulfid-Brücken enthalten oder heterologe, besonders bevorzugt eukaryontische Proteine, die in ihrer funktionellen Form als Di- oder Multimere vorliegen. Beispiele für eukaryontische Proteine sind Antikörper und deren Fragmente, Cytokine, Wachstumsfaktoren, Proteinkinasen sowie Proteinhormone.

Mittels des erfindungsgemäßen Verfahrens lassen sich auch heterologe Proteine, die in ihrer funktionellen Form als Di- oder Multimere vorliegen, d.h. eine Quartärstruktur besitzen und aus mehreren identischen (homologen) oder nicht-identischen (heterologen) Untereinheiten aufgebaut sind in hohen Ausbeuten in der korrekten aktiven Dimer- oder Multimer-Struktur aus dem Fermentationsmedium gewinnen, wenn dessen monomere Proteinketten mit Signalpeptiden für die Sekretion verknüpft sind und mittels des Sec-Systems in das Periplasma transportiert werden. Dies gelang sowohl mit Homo-Dimeren oder Multimeren, als auch bei Hetero-Dimeren oder -Multimeren, also bei Proteinen, bei denen sich die Proteinketten der Untereinheiten in ihrer Aminosäuresequenz unterscheiden. Besonders bevorzugt sind dabei Proteine, die aus mehreren unterschiedlichen Proteinketten aufgebaut sind, also Hetero-Dimere oder Hetero-Multimere darstellen. Dies war völlig unerwartet, da bei solchen Proteinen die einzelnen Proteinketten mittels des Sec-Systems zunächst unabhängig voneinander in das Periplasma transportiert werden müssen, um dort üblicherweise unter Mitwirkung von periplasmatischen Enzymen und Chaperonen in die richtige Sekundär-, Tertiär- und Quartärstruktur gefaltet bzw. assembliert zu werden. Hier ging der Fachmann bisher davon aus, dass die Freisetzung der Proteine in das Fermentationsmedium mit solchen komplexen Faltungs- und Assemblierungsvorgängen interferiert und eine Sekretion solcher Proteine in funktioneller Form daher besonders schwierig ist.

Eine besonders wichtige Klasse von Proteinen, die aus mehreren Proteinuntereinheiten besteht, sind Antikörper. Antikörper werden in der Forschung, in der Diagnostik und als Therapeutikum in großem Umfang eingesetzt, so dass besonders effiziente und in technischem Ausmaß mögliche Produktionsverfahren notwendig sind.

Bei Antikörpern wird zwischen Volllängen-Antikörpern und Antikörperfragmenten unterschieden. Volllängen-Antikörper bestehen aus vier Proteinketten, zwei identischen schweren Ketten und zwei identischen leichten Ketten. Die verschiedenen Ketten sind durch Disulfidbrücken miteinander verbunden. Jede schwere Kette ist aus einer variablen Region (V_{H}) und einer konstanten Region, welche die drei Domänen CH1, CH2 und CH3 umfasst, aufgebaut. Die Region der schweren Kette, die die Domänen CH2 und CH3 umfasst und die auch als Fc-Region bezeichnet wird, ist nicht an der Antigen-Bindung beteiligt, sondern hat andere Funktionen, wie z. B. die Aktivierung des Komplement-Systems. Jede leichte Kette ist aus einer variablen Region (V_{L}) und einer konstanten Region, die die Domäne C_{L} umfasst, aufgebaut. Abhängig von der Aminosäure-Sequenz der schweren Kette werden Antikörper (Immunglobuline) in fünf Klassen eingeordnet: IgA, IgD, IgE, IgG und IgM. Unter dem Begriff Volllängen-Antikörper sind alle Antikörper zu verstehen, bei denen die leichten Ketten jeweils die Domänen V_{L} und C_{L} umfassen und die schweren Ketten substantiell aus den Domänen V_{H}-CH1-CH2-CH3 aufgebaut sind und der Antikörper somit neben der Eigenschaft ein spezifisches Antigen binden zu können, auch in der Lage ist, andere Funktionen (z. B. die Aktivierung des Komplement-Systems) auszuführen.
Demgegenüber bestehen Antikörperfragmente aus lediglich einem Teil eines Volllängen-Antikörpers, üblicherweise aus dem Teil, der die Antigen-Bindestelle mit umfasst. Beispiele für Antikörperfragmente sind u.a. i) Fab-Fragmente, bei denen die leichten Ketten jeweils die Domänen V_{L} und C_{L} und die schweren Ketten jeweils die Domänen V_{H} und CH1 umfassen, ii) Fab'-Fragmente, die im Prinzip Fab-Fragmente darstellen, aber am C-Terminus der CH1-Domäne noch einen oder mehrere Cystein-Reste tragen oder iii) F(ab')₂-Fragmente, bei denen zwei Fab'-Fragmente mittels der Cystein-Reste am C-Terminus der CH1-Domäne über Disulfidbrücken miteinander verknüpft sind.

Zur Herstellung von Antikörperfragmenten wurde bereits E. coli verwendet, die Produktion fand dabei allerdings entweder im Cytoplasma oder im Periplasma statt. In beiden Fällen müssen die E. coli Zellen aufgeschlossen werden und die Antikörperfragmente von den restlichen E. coli Proteinen abgetrennt werden.

US6204023 und EP0396612 beschreiben die extrazelluläre Produktion von Fab-Fragmenten. Die Ausbeuten liegen im Bereich von wenigen Milligramm pro Liter. Better et al. (1993, Proc. Natl. Acad. Sci. USA 90, 457 - 61) beschreibt die extrazelluläre Herstellung von chimären Fab'- und F(ab')₂-AntikörperFragmenten mit dem E. coli-Stamm W3110ara⁻. Auch die dabei erzielten Ausbeuten von 200 - 700 mg/l sind für einen wirtschaftlichen Prozess im technischen Maßstab zu niedrig.

Volllängen-Antikörper können in E. coli bislang ausschließlich im Periplasma in der korrekten Quartärstruktur hergestellt werden (WO02/061090). Zur Gewinnung der Antikörper ist es dabei nötig, die Zellen aufzuschließen. Die Ausbeuten waren mit maximal 156 mg/l sehr niedrig. Höhere Ausbeute bis 880 mg wurden nur erreicht, wenn zusätzlich zu den Antikörper-Ketten, periplasmatische Faltungshelfer, wie die dsb-Proteine oder FkpA auf Plasmiden koexprimiert wurden. Der Antikörper musste aus der Vielzahl der anderen E. coli Proteinen aufgereinigt werden.

Bei Versuchen im Rahmen der vorliegenden Erfindung zeigte sich überraschend, dass bei Verwendung von E. coli lpp-Mutanten zur Herstellung von Antikörperfragmenten in technischem Maßstab extrazelluläre Ausbeuten von mehr als 1 g/l erreicht werden. Bevorzugte Antikörperfragmente sind dabei Fab, Fab' und F(ab')₂-Fragmente, besonders bevorzugt Fab-Fragmente.

Überraschend war ferner, dass auch bei der Herstellung von Volllängen-Antikörpern mittels des erfindungsgemäßen Verfahrens extrazelluläre, korrekt gefaltete und funktionelle Antikörper in hoher Ausbeute erhalten werden. Bevorzugte Volllängen-Antikörper sind dabei Antikörper der IgG- und IgM-Klasse, inbesonders der IgG-Klasse.

Für die Sekretion von Proteinen aus dem Cytoplasma in das Periplasma ist es notwendig, das 5'-Ende des Gens des zu produzierenden Proteins in frame mit dem 3'-Ende einer Signalsequenz für den Protein-Export zu verknüpfen. Dazu sind prinzipiell die Gene aller Signalsequenzen geeignet, die in E.coli eine Translokation des Zielproteins mit Hilfe des Sec-Apparats ermöglichen. Verschiedene Signalsequenzen sind im Stand der Technik beschrieben, so z. B. die Signalsequenzen der folgenden Gene: phoA, ompA, pelB, ompF, ompT, lamB, malE, Staphylococcal protein A, StII und andere (Choi & Lee, 2004). Bevorzugt sind die Signalsequenzen des phoA- und ompA-Gens von E. coli, besonders bevorzugt ist die Signalsequenz für eine Cyclodextrin-Glycosyltransferase (CGTase) aus Klebsiella pneumoniae M5a1 mit der Sequenz SEQ ID No. 3 (EP0448093).

Die Herstellung des DNS-Moleküls, das mindestens eine Fusion aus einer Signalsequenz und dem Gen des rekombinanten Zielproteins umfasst, erfolgt nach dem Fachmann bekannten Methoden.

So kann das Gen des Zielproteins zunächst mittels PCR unter Verwendung von Oligonukleotiden als Primer amplifiziert und anschließend mit gängigen molekularbiologischen Techniken mit dem DNS-Molekül, das die Sequenz eines Signalpeptids umfasst und das auf analoge Weise wie das Gen des Zielproteins erzeugt wurde, derart verknüpft werden, dass eine in frame-Fusion, d.h. ein durchgehender Leserahmen umfassend die Signalsequenz und das Gen des Zielproteins, entsteht. Alternativ kann auch das gesamte DNS-Molekül, das beide oben genannten funktionellen Abschnitte umfasst, mittels Gensynthese hergestellt werden. Diese Signalsequenz-rekombinante Gen-Fusion kann dann entweder in einen Vektor, z. B. ein Plasmid, eingebracht werden, oder direkt nach bekannten Methoden in das Chromosom der Wirtszelle integriert werden. Bevorzugt wird die Signalsequenz-rekombinante Gen-Fusion in Plasmide eingebracht.

Für die Sekretion eines Proteins, das aus mehreren unterschiedlichen Untereinheiten besteht, aus dem Cytoplasma in das Periplasma ist es notwendig, das 5'-Ende des jeweiligen Gens der zu produzierenden Untereinheit (Zielgen) in frame mit dem 3'-Ende einer Signalsequenz für den Protein-Export zu verknüpfen. Dabei können die Gene der verschiedenen Untereinheiten mit unterschiedlichen Signalsequenzen verknüpft werden oder der gleichen. Bevorzugt ist die Verknüpfung mit unterschiedlichen Signalsequenzen, besonders bevorzugt ist die Verknüpfung der einen Untereinheit mit der Signalsequenz des phoA- oder ompA-Gens von E. coli und die Verknüpfung der zweiten Untereinheit mit der Signalsequenz für eine Cyclodextrin-Glycosyl-transferase (CGTase) aus Klebsiella pneumoniae mit der Sequenz SEQ ID No. 3 (EP0448093).

Die Signalsequenz-Zielgen-Fusionen der einzelnen Untereinheiten können dann entweder in einen Vektor, z. B. ein Plasmid, eingebracht werden, oder direkt nach bekannten Methoden in das Chromosom der Wirtszelle integriert werden. Dabei können die Signalsequenz-Zielgen-Fusionen der einzelnen Untereinheiten auf getrennten, aber miteinander kompatiblen Plasmiden kloniert werden oder sie können auf einem Plasmid kloniert werden. Die Genfusionen können dabei in einem Operon zusammengefasst werden oder sie können in jeweils separaten Cistronen exprimiert werden. Bevorzugt ist hier ein Zusammenfassen in einem Operon. Genauso können die beiden Genkonstrukte in einem Operon zusammengefasst oder in jeweils separaten Cistronen in das Chromosom der Wirtszelle integriert werden. Bevorzugt ist auch hier ein Zusammenfassen in einem Operon.

Vorzugsweise wird das DNS-Expressionskonstrukt aus einer Signalsequenz und einem rekombinanten Gen kodierend das zu sekretierende Protein mit in E. coli funktionellen Expressionssignalen versehen (Promotor, Transkriptions-, Translationsstart, Ribosomenbindestelle, Terminator).

Als Promotoren eignen sich alle dem Fachmann bekannten Promotoren, wie einerseits beispielsweise induzierbare Promotoren wie der lac-, tac-, trc-, lambda PL, ara- oder tet-Promotor oder davon abgeleitete Sequenzen. Andererseits kann auch eine dauerhafte Expression erfolgen durch Verwendung eines konstitutiven Promotors, wie z. B. des GAPDH-Promotors. Es kann aber auch der normalerweise mit dem Gen des zu produzierenden rekombinanten Proteins verknüpfte Promotor Verwendung finden.

Dieses Expressionskonstrukt (Promoter-Signalsequenzrekombinantes-Gen) für das zu produzierende Protein wird danach unter Anwendung von dem Fachmann bekannten Methoden in die Zellen mit lpp-Mutation eingebracht.

Dies erfolgt z. B. auf einem Vektor, z. B. einem Plasmid, wie etwa einem Abkömmling von bekannten Expressionsvektoren, wie pJF118EH, pKK223-3, pUC18, pBR322, pACYC184, pASK-IBA3 oder pET. Als Selektionsmarker für Plasmide geeignet sind Gene, die für eine Resistenz gegenüber z. B. Ampicillin, Tetracyclin, Chloramphenicol, Kanamycin oder andere Antibiotika codieren.

Erfindungsgemäß wird somit vorzugsweise ein E. coli Stamm eingesetzt, in dem das rekombinante Gen funktionell verknüpft mit einer Signalsequenz kodierend für ein in E. coli aktives Signalpeptid, vorzugsweise ferner mit in E. coli funktionellen Expressionssignalen, vorzugsweise einem Promotor, einem Transkriptions-, Translationsstart, einer Ribosomenbindestelle, und einem Terminator versehen ist. Es handelt sich dabei vorzugsweise um die bereits vorstehend genannten Expressionssignale.

Die Kultivierung (Fermentation) der mit einem Expressionsplasmid transformierten Zellen erfolgt im technischen Maßstab nach üblichen, dem Fachmann bekannten Fermentationsverfahren in einem Bioreaktor (Fermenter).

Die Fermentation findet vorzugsweise in einem üblichen Bioreaktor, beispielsweise einem Rührkessel, einem Blasensäulen-Fermenter oder einem Airlift-Fermenter statt. Besonders bevorzugt ist ein Rührkessel-Fermenter. Bevorzugt sind Fermenter mit einem Volumen 1, von > 50 l.

Bei der Fermentation werden die Zellen des Protein-Produktionsstammes in einem Flüssigmedium über einen Zeitraum von 16 - 150 h kultiviert, wobei verschiedene Parameter, wie z. B. die Nährstoff-Zufuhr, der Sauerstoff-Partialdruck, der pH-Wert und die Temperatur der Kultur laufend kontrolliert und genau gesteuert werden. Der Zeitraum der Kultivierung beträgt vorzugsweise 24 - 72 h.

Als Fermentationsmedien kommen in Prinzip alle gängigen, dem Fachmann bekannten Medien für die Kultivierung von Mikroorganismen in Frage.

Dabei können Komplexmedien oder Minimalsalzmedien, denen ein bestimmter Anteil von Komplex-Komponenten, wie z. B. Pepton, Trypton, Hefeextrakt, Melasse oder Corn Steep liquor zugesetzt wird, benützt werden. Bevorzugt sind dabei Medien, die Ca²⁺-Ionen in einer Konzentration von 10 mg/l bis 5000 mg/l, besonders bevorzugt 40 mg/l - 5000 mg/l enthalten, und Mg²⁺-Ionen in einer Konzentration größer 48 mg/l bis maximal 5000 mg/l enthalten.

Bevorzugt für die Herstellung von Pharmaproteinen (pharmazeutisch aktive Proteine) sind chemisch definierte Salzmedien, also Medien, die im Gegensatz zum Vollmedium eine genau definierte Substrat-Zusammensetzung besitzen. Es ist bekannt, dass empfindliche Mikroorganismen in solchen Medien nicht oder verlangsamt wachsen. Es war daher unerwartet und überraschend, dass ein E. coli Stamm enthaltend eine lpp-Mutation sowie ein Gen kodierend ein heterologes Protein, welches in frame mit einer Signalsequenz kodierend für ein in E. coli funktionelles Signalpeptid verbunden ist, in einem definierten Salzmedium, das Ca²⁺- und Mg²⁺-Ionen enthält, wächst und große Mengen des heterologen Proteins in das Salzmedium sekretiert.

In diesem Verfahren wächst der E. coli Stamm enthaltend eine lpp-Mutation sowie ein Gen kodierend ein heterologes Protein, welches in frame mit einer Signalsequenz kodierend für ein in E. coli funktionelles Signalpeptid verbunden ist, in einer zu einem Stamm ohne lpp-Mutation vergleichbar kurzen Fermentationszeit zu vergleichbar hohen Zelldichten heran und sekretiert dabei große Mengen des heterologen Proteins in das Salzmedium. Besonders bevorzugt sind dabei Salzmedien, die Ca²⁺-Ionen in einer Konzentration von 10 mg/l bis 5000 mg/l, besonders bevorzugt 40 - 5000 mg/l enthalten, und Mg²⁺-Ionen in einer Konzentration größer 48 mg/l bis maximal 5000 mg/l enthalten.

Als primäre Kohlenstoffquelle für die Fermentation können prinzipiell alle von den Zellen verwertbaren Zucker, Zuckeralkohole oder organische Säuren bzw. deren Salze verwendet werden. Dabei werden bevorzugt Glucose, Laktose oder Glycerin eingesetzt. Besonders bevorzugt sind Glucose und Laktose. Auch ist eine kombinierte Fütterung mehrerer verschiedener Kohlenstoffquellen möglich. Die Kohlenstoffquelle kann dabei zu Beginn der Fermentation vollständig im Fermentationsmedium vorgelegt werden oder es wird nichts oder nur ein Teil der Kohlenstoffquelle zu Beginn vorgelegt und die Kohlenstoffquelle wird über den Verlauf der Fermentation zugefüttert. Besonders bevorzugt ist dabei eine Ausführungsform, bei der ein Teil der Kohlenstoffquelle vorgelegt wird und ein Teil gefüttert wird. Besonders bevorzugt wird die Kohlenstoffquelle in einer Konzentration von 10 - 30 g/l vorgelegt, die Fütterung gestartet, wenn die Konzentration auf kleiner 5 g/l abgefallen ist und so gestaltet, dass die Konzentration unter 5 g/l gehalten wird.

Der Sauerstoff-Partialdruck (pO₂) in der Kultur beträgt vorzugsweise zwischen 10 und 70 % Sättigung. Bevorzugt wird ein pO₂ zwischen 30 und 60 %, besonders bevorzugt liegt der pO₂ zwischen 45 und 55 % Sättigung.

Der pH-Wert der Kultur liegt vorzugsweise zwischen pH 6 und pH 8. Bevorzugt wird ein pH-Wert zwischen 6,5 und 7,5 eingestellt, besonders bevorzugt wird der pH-Wert der Kultur zwischen 6,8 und 7,2 gehalten.

Die Temperatur der Kultur beträgt vorzugsweise zwischen 15 und 45°C. Bevorzugt ist ein Temperaturbereich zwischen 20 und 40°C, besonders bevorzugt ist ein Temperaturbereich zwischen 25 und 35°C, ganz besonders bevorzugt sind 30°C.

Unter den genannten Bedingungen wachsen E. coli Stämme enthaltend eine lpp-Mutation sowie ein Gen kodierend ein heterologes Protein, welches in frame mit einer Signalsequenz kodierend für ein in E. coli funktionelles Signalpeptid verknüpft ist, in kurzer Fermentationszeit im Produktionsmaßstab, d.h. in einem Fermenter mit einem Arbeitsvolumen von > 5 1, zu normalen Zelldichten heran und sekretieren dabei große Mengen an heterologen Proteinen in das Fermentationsmedium.

Die Aufreinigung des sekretierten Proteins aus dem Rohprodukt kann durch übliche und dem Fachmann bekannte Aufreinigungsmethoden geschehen, wie sie im Stand der Technik bekannt sind. Üblicherweise werden in einem ersten Schritt durch Separationsmethoden, wie Zentrifugation oder Filtration, die Zellen von dem sekretierten Zielprotein abgetrennt. Das Zielprotein kann dann z. B. durch Ultrafiltration aufkonzentriert werden und wird dann über Standardmethoden, wie Fällung, Chromatographie oder Ultrafiltration weiter gereinigt. Besonders bevorzugt sind hierbei Methoden, wie Affinitätschromatographie, bei der die bereits korrekt gefaltete native Konformation des Proteins ausgenützt wird.
Fig. 1 zeigt den Vektor pK03-lpp1 aus Bsp. 2.
Fig. 2 zeigt den Vektor pK03-lpp3 aus Bsp. 3.
Fig. 3 zeigt den Klonierungsvektor pJF118ut aus Bsp. 4.
Fig. 4 zeigt das CGTase-Expressionsplasmid pCGT aus Bsp. 4.
Fig. 5 zeigt das Interferonα2b-Expressionsplasmid pIFN aus Bsp. 6.
Fig. 6 zeigt das Plasmid pHC-Anti-Lysozym aus Bsp. 7.
Fig. 7 zeigt das Fab-Expressionsplasmid pFab-Anti-Lysozym aus Bsp. 7.
Fig. 8 zeigt das Plasmid pHC-Anti-TF aus Bsp. 8.
Fig. 9 zeigt das Anti-TF-Antikörper-Expressionsplasmid pAK-Anti-TF aus Bsp. 8.

Die in den Figuren verwendeten Abkürzungen haben die folgende Bedeutung:
- tac p/o:: tac-Promotor/Operator;
- cmR:: Chloramphenicol-Resistenz;
- lpp1:: lpp1-Allel mit Basensubstitution, die zum Amino- säure-Austausch Arg77Cys führt (R77C);
- lpp3:: lpp3-Allel mit Basensubstitution, die zum Amino- säure-Austausch Gly14Asp führt (G14D);
- M130ri:: M13-Replikationsursprung;
- sacB:: Laevansucrase-Gen aus Bacillus;
- repA:: pSC101-Replikationsursprung, temperatursensitiv;
- rrnB:: Terminator;
- bla:: β-Lactamase-Gen (Ampicillin-Resistenz);
- ColE1:: ColE1-Replikationsursprung;
- TcR:: Tetracyclin-Resistenz-Gen;
- lacIq:: Repressor des tac-Promotors;
- cgt-SP:: Signalpeptid der CGTase;
- CGTase:: CGTase-Gen;
- SD:: Shine-Dalgarno-Sequenz;
- IFNalpha2b:: Interferonα2b-Gen;
- ompA-SP:: ompA-Signalpeptid;
- (VH)-CH1:: Leserahmen für das Fragment der schweren Kette mit den Domänen VH und CH1 und C-terminalem His- Tag ;
- (VL)-CL:: Leserahmen für das Fragment der leichten Kette mit den Domänen VL und CL;
- His-Tag:: His-Tag am C-Terminus der schweren Kette des Fab-Fragments;
- HC (Anti-TF):: Leserahmen der schweren Kette des Anti-TF- Antikörpers;
- LC (Anti-TF):: Leserahmen der leichten Kette des Anti-TF- Antikörpers.

Die folgenden Beispiele dienen der weiteren Erläuterung der Erfindung.

Sämtliche eingesetzten molekularbiologischen Verfahren, wie Polymerase-Ketten-Reaktion (PCR), Gensynthese, Isolierung und Reinigung von DNS, Modifikation von DNS durch Restriktionsenzyme, Klenow-Fragment und Ligase, Transformation etc. wurden in der dem Fachmann bekannten, in der Literatur beschriebenen oder von den jeweiligen Herstellern empfohlenen Art und Weise durchgeführt.

### Beispiel 1: Erzeugung einer chromosomalen lpp-Deletionsmutante aus einem E. coli Wildtyp-Stamm

Zur Erzeugung einer *lpp*-Deletionsmutante des *E*. *coli* Wildtyp-Stammes W3110 (American Type Culture Collection (ATCC): 27325) mit Hilfe der λ-Rekombinase wurde nach der Methode von Datsenko und Wanner (2000, Proc. Natl. Acad. Sci. U S A. 97: 6640 - 5) vorgegangen. Dabei wurde zunächst mit Hilfe der Polymerase-Kettenreaktion (PCR) unter Verwendung der Oligonukleotide lpp1 (SEQ ID No. 4) und lpp2 (SEQ ID No. 5) als Primern und dem Plasmid pKD3 (Coli Genetic Stock Center (CGSC): 7631) als Matrize ein lineares DNS-Fragment erzeugt, welches ein Chloramphenicol-Resistenzgen enhält, und das von jeweils 50 Basenpaaren der upstream- bzw. der downstream-Region des *lpp*-Gens flankiert ist.
Der Stamm W3110 wurde zuerst mit dem Plasmid pKD46 (CGSC: 7739) transformiert. Kompetente Zellen des so erhaltenen Stammes W3110 pKD46, welche nach den Angaben von Datsenko und Wanner hergestellt worden waren, wurden mit dem per PCR erzeugten linearen DNS-Fragment transformiert. Die Selektion auf Integration der Chloramphenicol-Resistenzkassette in das Chromosom von W3110 an der Position des *lpp*-Gens erfolgte auf LB-AgarPlatten, die 20 mg/l Chloramphenicol enthielten. Auf diese Weise wurden Zellen erhalten, bei denen das *lpp*-Gen nahezu vollständig durch die Chloramphenicol-Resistenzkassette ersetzt worden war. Dass die Integration an der korrekten Position im Chromosom erfolgte, wurde mittels PCR unter Verwendung der Oligonukleotide pykF (SEQ ID No. 6) und ynhG2 (SEQ ID No. 7) und chromosomaler DNS der Chloramphenicol-resistenten Zellen als Matrize bestätigt.
Die Zellen wurden von dem Plasmid pKD46 nach der beschriebenen Prozedur (Datsenko und Wanner) kuriert und der auf diese Weise erzeugte Stamm wurde mit W3110*lpp*::cat bezeichnet. Die Entfernung der Chloramphenicol-Resistenzkassette aus dem Chromosom des Stammes W3110*lpp*::cat erfolgte nach der Vorschrift von Datsenko und Wanner mit Hilfe des Plasmids pCP20 (CGSC: 7629), welches das FLP-Rekombinase-Gen trägt. Die schließlich mit dieser Prozedur erhaltene Chloramphenicolsensitive *lpp*-Deletionsmutante von W3110 wurde mit W3110Δlpp bezeichnet.

### Beispiel 2: Erzeugung einer chromosomalen lpp1-Mutante aus einem E. coli Wildtyp-Stamm

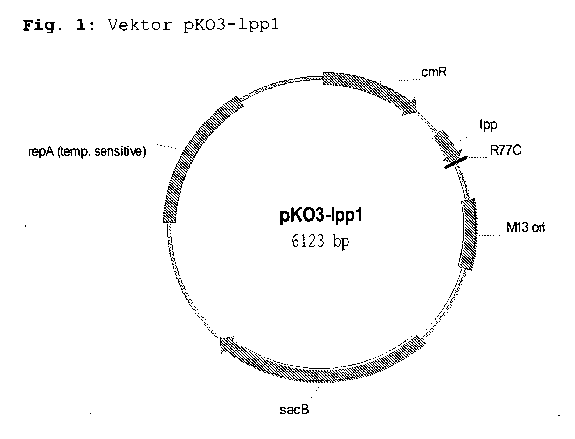

Der Austausch des lpp-Wildtypgens im Chromosom des Stammes W3110 gegen das lpp1-Allel erfolgte über homologe Rekombination. Dazu wurde folgendermaßen vorgegangen:
Mittels Gensynthese wurde ein DNS-Molekül hergestellt, welches das lpp1-Allel sowie ca. 200 Basenpaare der 3'-seitig des lpp-Wildtypgens gelegenen DNS-Region enthält (SEQ ID No. 8). Außerdem trägt dieses DNS-Molekül an beiden Enden jeweils eine Schnittstelle für das Restriktionsenzym BamHI. Das lpp1-Allel umfasst die Basen 9 bis 245 von SEQ ID No. 8. Im Unterschied zum lpp-Wildtypgen (SEQ ID No. 1) weist das lpp1-Allel eine Basensubstitution an Position 229 (C zu T) des lpp-Gens auf, was zu einem Austausch des Arginin-Rests an Position 77 gegen einen Cystein-Rest im unprozessierten Lpp-Protein führt.
Das per Gensynthese erzeugte DNS-Molekül mit der SEQ ID No. 8 wurde vollständig mit dem Restriktionsenzym BamHI geschnitten. Der Klonierungsvektor pK03 (Link et al., 1997, J. Bacteriol. 179, 6228 - 37; Harvard Medical School, Department of Genetics, 200 Longwood Ave, Boston, MA 02115) wurde zunächst ebenfalls mit dem Restriktionsenzym BamHI geschnitten. Das auf diese Weise linearisierte Plasmid wurde anschließend mit Alkalischer Phosphatase behandelt, um später eine Religation des Vektors zu verhindern. Die beiden auf diese Weise geschnittenen DNS-Moleküle wurden miteinander ligiert. Das so erzeugte Plasmid wurde mit pK03-lpp1 bezeichnet (Fig. 1).
Der Stamm W3110 wurde mittels der CaCl₂-Methode mit dem Plasmid pK03-lpp1 transformiert, wobei mit Ampicillin auf plasmidtragende Zellen selektiert wurde. Der anschließende Austausch des lpp-Wildtypgens gegen das lpp1-Allel erfolgte über den Mechanismus der homologen Rekombination nach der in Link et al. (1997) beschriebenen Prozedur. Dass dieser Austausch basengenau an der richtigen Position im Chromosom erfolgte, wurde dadurch überprüft, dass die chromosomale lpp-Region mittels PCR unter Verwendung der Oligonukleotide pykF (SEQ ID No. 6) und ynhG2 (SEQ ID No. 7) und chromosomaler DNS der vermeintlichen lpp1-Mutante als Matrize erst amplifiziert und anschließend das PCR-Produkt mit denselben Oligonukleotiden sequenziert wurde.
Die schließlich auf diese Weise erzeugte lpp1-Mutante von W3110 wurde mit W31101pp1 bezeichnet.

### Beispiel 3: Erzeugung einer chromosomalen lpp3-Mutante aus einem E. coli Wildtyp-Stamm

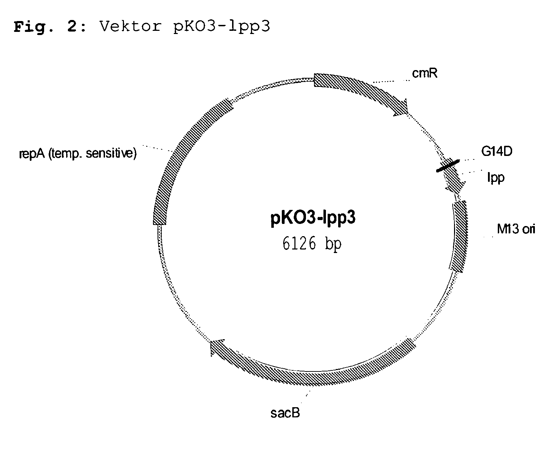

Bei der Erzeugung einer chromosomalen lpp3-Mutante von W3110, welche wie die lpp1-Mutante nur eine Punktmutation im lpp-Gen aufweist, wurde analog zu Beispiel 2 vorgegangen, mit dem Unterschied, dass anstelle des DNS-Fragments mit der SEQ ID No. 8 ein DNS-Molekül mit der SEQ ID No. 9, welches ebenfalls per Gensynthese hergestellt worden war, verwendet wurde. Dieses DNS-Molekül enthält das lpp3-Allel (Basen 211 bis 447) sowie ca. 200 Basenpaare der 5'-seitig des lpp-Wildtypgens gelegenen DNS-Region. Außerdem trägt dieses DNS-Molekül an beiden Enden jeweils eine Schnittstelle für das Restriktionsenzym BamHI. Das lpp3-Allel weist im Unterschied zur SEQ ID No. 1 eine Basensubstitution an Position 41 (G zu A) des lpp-Gens auf, was zu einem Austausch des Glycin-Rests an Position 14 gegen einen Asparaginsäure-Rest im noch unprozessierten Lpp-Protein führt. Das durch Ligation der jeweils mit BamHI geschnittenen DNS-Fragmente von Plasmid pK03 und dem lpp3-Allel haltigen DNS-Molekül erzeugte Plasmid pK03-lpp3 (Fig. 2) wurde in den Stamm W3110 wie oben beschrieben transformiert. Mittels der Prozedur von Link et al. wurde schließlich der Stamm W3110lpp3 erhalten. Die Stammüberprüfung erfolgte wie in Beispiel 2 beschrieben.

### Beispiel 4: Fermentative Produktion einer Cyclodextrin-Glycosyl-Transferase mit lpp-Mutanten im 10 l-Maßstab

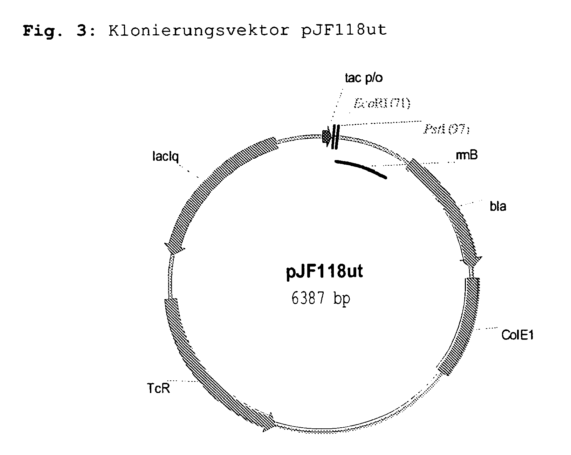

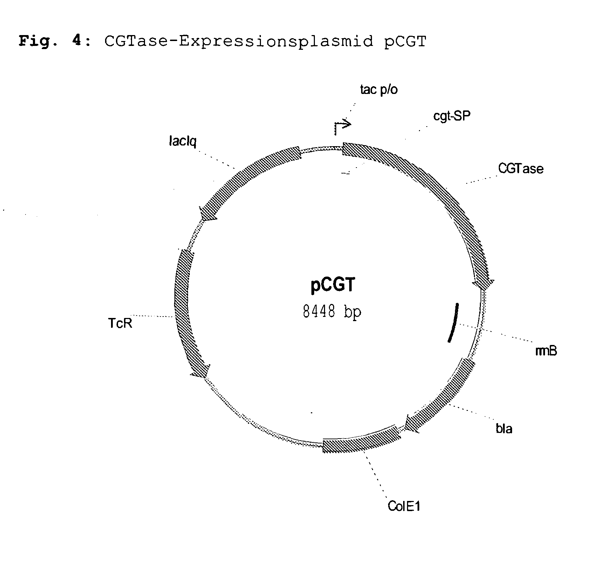

Ein DNS-Fragment mit der SEQ ID No. 10, welches ein Cyclodextrin-Glycosyl-Transferase (CGTase)-Gen aus Klebsiella pneumoniae M5a1 enthält (Genbanknr. M15264), wurde per Gensynthese hergestellt. Dieses DNS-Fragment wurde in den Expressionsvektor pJF118ut (Fig. 3) kloniert, welcher bei der DSMZ-Deutsche Sammlung von Mikroorganismen und Zellkulturen GmbH (Braunschweig) unter der Nummer DSM 18596 hinterlegt ist. pJF118ut ist ein Derivat des bekannten Expressionsvektors pKK223-3 (Amersham Pharmacia Biotech) und enthält neben dem β-Lactamase-Gen und dem Tetracyclin-Resistenzgen noch den tac-Promotor, der durch das LacIq-Genprodukt, dessen Gen ebenfalls auf dem Plasmid vorliegt, reprimiert wird und der durch einen Induktor, wie z. B. D-Lactose oder Isopropyl-β-D-thiogalactopyranosid (IPTG), angeschaltet werden kann.
Das Plasmid pJF118ut wurde mit dem Restriktionsenzym EcoRI vollständig geschnitten und die jeweils an den 5'-Enden des linearen DNS-Fragments überstehenden Basen wurden mit S1-Nuklease abgespalten. Das auf diese Weise vorbereitete Vektor-DNS-Molekül wurde mit dem CGTase-umfassenden DNS-Fragment (SEQ ID No. 10) unter Verwendung der T4-Ligase ligiert. Der Stamm DH5α wurde mit dem Ligationsansatz nach der CaCl₂-Methode transformiert, wobei mittels Ampicillin (100 mg/l) auf plasmidhaltige Zellen selektiert wurde. Aus Ampicillin-resistenten Transformanden wurde das Plasmid wieder isoliert und durch Restriktionsanalyse überprüft. Das auf diese Weise erzeugte Plasmid, bei dem die Expression des CGTase-Gens unter der Kontrolle des tac-Promotors liegt, wurde mit pCGT bezeichnet (Fig. 4).

Für die Produktion einer Cyclodextrin-Glycosyl-Transferase im 10 1-Maßstab wurden die Stämme W3110Δlpp, W3110lpp1 und W3110lpp3 jeweils mit dem Plasmid pCGT mittels der CaCl₂-Methode transformiert. Die Selektion auf plasmidhaltige Zellen erfolgte mittels Ampicillin (100 mg/l).

Die Produktion wurde in 10 1 Rührkesselfermentern durchgeführt.
Der mit 6 1 des Fermentationsmediums FM4 (1,5 g/l KH₂PO₄; 5 g/l (NH₄)₂SO₄; 0,5 g/l MgSO₄ x 7 H₂O; 0,15 g/l CaCl₂ x 2 H₂O, 0,075 g/l FeSO₄ x 7 H₂O; 1 g/l Na₃Citrat x 2 H₂O; 0,5 g/l NaCl; 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O); 5 mg/l Vitamin B₁; 3 g/l Phyton; 1,5 g/l Hefeextrakt; 10 g/l Glukose; 100 mg/l Ampicillin) befüllte Fermenter wurde im Verhältnis 1:10 mit einer Vorkultur, die über Nacht im gleichen Medium kultiviert wurde, angeimpft. Während der Fermentation wurde eine Temperatur von 30°C eingestellt und der pH-Wert durch Zudosierung von NH₄OH bzw. H₃PO₄ bei einem Wert von 7,0 konstant gehalten. Glukose wurde über die Fermentation hinweg zudosiert, wobei eine maximale Glukose-Konzentration im Fermentationsmedium von < 10 g/l angestrebt wurde. Die Induktion der Expression erfolgte durch Zugabe von Isopropyl-β-D-thiogalactopyranosid (IPTG) ad 0,1 mM am Ende der logarithmischen Wachstumsphase.

Nach 72 h Fermentation wurden Proben entnommen, die Zellen durch Zentrifugation vom Fermentationsmedium abgetrennt und der CGTase-Gehalt im Fermentationsüberstand durch folgenden Aktivitätstest bestimmt:
Testpuffer: 5 mM Tris-HCl-Puffer > pH 6,5, 5 mM CaSO₄ . 2 H₂0 Substrat: 10 %ige Noreduxlösung in Test-Puffer (pH 6,5) Testansatz: 1 ml Substratlösung + 1 ml abzentrifugierter und
gegebenenfalls verdünnter Kulturüberstand (5 Min., 12 000 rpm) + 3 ml Methanol
Reaktionstemperatur: 40°C

Enzymtest:
◇ Vortemperieren der Lösungen (ca. 5 Min. bei 40°C)
◇ Zugabe der Enzymlösung zur Substratlösung; rasches Mischen (Whirl-Mixer)
◇ Inkubation für 3 Min. bei 40°C
◇ Stoppen der Enzymreaktion durch Methanol-Zugabe; rasches Mischen (Whirl-Mixer)
◇ Abkühlen des Ansatzes auf Eis (ca. 5 Min.)
◇ Abzentrifugieren (5 Min., 12 000 rpm) und Abpipettieren des klaren Überstandes
◇ Analyse des produzierten CDs mittels HPLC Enzymaktivität: A = G*V1*V2/(t*MG) (Units/ml)
A = Aktivität
G = Gehalt an CD in mg/l = Testansatz: Flächeneinheiten x 104 / Standardlösung (10mg/ml)/ Flächeneinheiten
V1 = Verdünnungsfaktor im Testansatz (bei Durchführung nach o.g. Angaben: V1 = 5)
V2 = Verdünnungsfaktor des Kulturüberstands vor dem Einsatz im Test; wenn unverdünnt: V2 = 1
t = Reaktionszeit in Min.
MG = Molekulargewicht in g/mol (CD = 973 g/mol) 1 Unit = 1µMol Produkt / Min.

Aus der auf diese Weise bestimmten CGTase-Aktivität lässt sich die Menge der im Fermentationsüberstand vorhandenen CGTase berechnen. Dabei entsprechen 150 U/ml CGTase-Aktivität etwa 1 g/l CGTase-Protein.

Tabelle 1 zeigt die jeweils erzielten Cyclodextrin-Glycosyltransferase-Ausbeuten.

**Tabelle 1: Cyclodextrin-Glycosyltransferase-Ausbeuten im Fermentationsüberstand nach 72 Fermentation**

| Stamm | Cyclodextrin-Glycosyl-transferase (U/ml) | Cyclodextrin-Glycosyl-transferase (g/l) |
|---|---|---|
| W3110Δlpp/pCGT | 480 | 3,2 |
| W3110lpp1/pCGT | 450 | 3,0 |
| W3110lpp3/pCGT | 520 | 3,5 |

### Beispiel 5: Fermentative Produktion eines Hirudin-Derivats mit lpp-Mutanten im 10 l-Maßstab

Hirudin ist ein Polypeptid mit 65 Aminosäuren und wurde ursprünglich aus dem Blutegel *Hirudo medicinalis* isoliert. In diesem Beispiel wird die fermentative Herstellung eines Hirudin-Derivats mit der N-terminalen Aminosäure-Sequenz Ala-Thr-Tyr-Thr-Asp beschrieben.

Für die Produktion des Hirudin-Derivats wurden die Stämme W3110Δlpp, W3110lpp1 und W3110lpp3 zunächst jeweils mit dem Plasmid pCMT203 mittels der CaCl₂-Methode transformiert. Die Selektion auf plasmidhaltige Zellen erfolgte mittels Ampicillin (100 mg/l).

Das Plasmid pCMT203 enthält neben dem Gen für die Resistenz gegen Ampicillin u.a. auch das Strukturgen des Hirudin-Derivats, welches *in frame* an das 3'-Ende einer CGTase-Signalsequenz anfusioniert ist. Die Expression der CGTase-Signalsequenz-Hirudin-Fusion steht unter der Kontrolle des tac-Promotors. Das Plasmid pCMT203 ist in EP0448093B1 beschrieben.

Die Produktion des Hirudin-Derivats im 10 1-Maßstab erfolgte analog dem in Beispiel 4 beschriebenen Verfahren mit den Stämmen W3110Δlpp/pCMT203, W3110lpp1/pCMT203 und W3110lpp3/pCMT203. Nach 72 h Fermentation wurden Proben entnommen, anschließend die Zellen durch Zentrifugation vom Fermentationsmedium abgetrennt und der Hirudin-Gehalt im Fermentationsüberstand mittels eines Thrombin-Inaktiverungstests, wie in Chang (1991, J. Biol. Chem. 266, 10839 - 43) beschrieben, bestimmt. Dabei entsprechen 16000 Antithrombin(AT)-Units/ml Hirudin-Aktivität etwa 1 g/l Hirudin-Protein. Tabelle 2 zeigt die jeweils erzielten Hirudin-Ausbeuten im Fermentationsüberstand.

**Tabelle 2: Hirudin-Ausbeute (in AT-U/ml und g/l) im Fermentationsüberstand nach 72 h Fermentation.**

| Stamm | Hirudin (AT-U/ml) | Hirudin (g/l) |
|---|---|---|
| W3110Δlpp/pCMT203 | 50000 | 3,1 |
| W3110lpp1/pCMT203 | 44000 | 2,8 |
| W3110lpp3/pCMT203 | 48000 | 3,0 |

### Beispiel 6: Fermentative Produktion von Interferonα2b mit lpp-Mutanten im 10 l-Maßstab

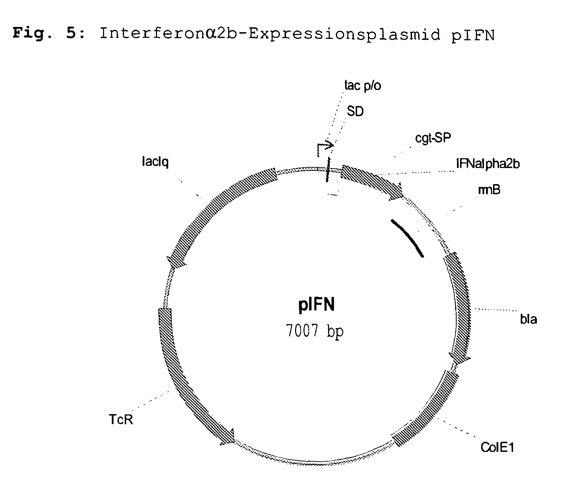

Ein weiteres pharmazeutisch interessantes Protein, das mit Hilfe einer *lpp*-Mutante von *E*. *coli* extrazellulär hergestellt werden kann, ist das Interferonα2b.
Bei der Erzeugung des Expressionsvektors für das Interferonα2b-Gen wurde wie folgt vorgegangen:
Ein DNS-Fragment mit der SEQ ID No. 11, welches eine Genfusion bestehend aus der in EP 0220714 beschriebenen CGTase-Signalsequenz (SEQ ID No. 3) und dem Gen für Interferonα2b enthält, wurde per Gensynthese hergestellt.
Dieses DNS-Fragment wurde mit den Restriktionsenzymen EcoRI und PstI geschnitten und mit dem Expressionsvektor pJF118ut, der mit denselben Restriktionsenzymen geschnitten worden war, ligiert. Das aus dieser Klonierung resultierende Plasmid, bei dem die Expression des Interferonα2b-Gens unter der Kontrolle des tac-Promotors liegt, wurde mit pIFN (Fig. 5) bezeichnet.

Für die Herstellung von Interferonα2b wurden die Stämme W3110Δlpp, W3110lpp1 und W3110lpp3 jeweils mit dem Plasmid pIFN nach der CaCl₂-Methode transformiert. Die Selektion auf plasmidhaltige Zellen erfolgte mittels Ampicillin (100 mg/l).

Die Produktion von Interferonα2b im 10 1-Maßstab erfolgte analog dem in Beispiel 4 beschriebenen Verfahren mit den Stämmen W3110Δlpp/pIFN, W3110lpp1/pIFN und W3110lpp3/pIFN. Nach 72 h Fermentation wurden Proben entnommen, anschließend die Zellen durch Zentrifugation vom Fermentationsmedium abgetrennt und der Interferona2b-Gehalt im Fermentationsüberstand bestimmt. Dazu wurden die Proteine im Fermentationsüberstand elektrophoretisch in einem SDS-Polyacrylamidgel aufgetrennt und mittels Detektion im Immunoblot mit Anti-Interferon-spezifischen Antikörpern wie folgt quantifiziert:
1 µl Überstand wurde mit Probenpuffer versetzt (2x Tris SDS - Sample Buffer (Invitrogen Cat.No. LC2676): 0.125 M Tris.HCl, pH 6.8, 4 % w/v SDS, 20 % v/v Glycerin, 0.00 5% v/v Bromphenolblau, 5 % beta-Mercaptoethanol). Außerdem wurden definierte Mengen von Interferonα2b als Standard mit aufgetragen. Denaturieren der Proteine erfolgte durch Erhitzen auf 100°C für 5 Min., Abkühlen für 2 Min. auf Eis und abzentrifugieren.
Die Proteine wurden durch Elektrophorese in einem 12 % NuPAGE^{®} Bis-Tris-Gel (Invitrogen Cat.No. NP0341) mit 1x MES-haltigem Running Buffer (Invitrogen Cat.No. NP0002) aufgetrennt (Elektrophorese-Parameter: 40 Min. bei 200 V).
Detektion und Quantifizierung über Immunoblot wurde nach folgender Vorschrift durchgeführt:
Transfer im Naßblot-Verfahren:
   Modul: Amersham: Hoefer TE 22 Mini Tank Transfer Unit, Code Number: 80-6204-26
   Membran: Nitrocellulose-Membran (Schleicher&Schuell, BA 85, Cellulosenitrat (E), 0,45 µm Porengröße)
   Whatman-Filter und Nitrocellulose-Membran in passende Größe schneiden und mit Schaumstoffstücken (Schwämmen) in Transferpuffer (Invitrogen Cat.No. LC3675) luftblasenfrei tränken. Aufbau des Sandwiches: schwarzes Gitter, Verbindung mit der Kathode, 2 Schwämme, je 3 mm dick, Whatman-Papier, SDS-Polyacrylamidgel, NC-Membran, Whatman, 1 Schwamm, 6 mm dick, weißes Gitter, Verbindung mit der Anode.
   Transferbedingungen: I = 200mA constant current, U = unbegrenzt, Laufzeit 60 Min.
Prehybridisierung
   Inkubation der Membran in 25 ml Prehybridisierungspuffer 30 min bei RT schwenken
Hybridisierung 1. Antikörper
   Inkubation der Membran in 25 ml Prehybridisierungspuffer + 0,15 µg/ml (-> 3,75 µg) Anti-human-IFN-alpha Antibody (Pepro Tech EC, über Biozol Cat. No.: 500-P32A)
   90 Min. oder über Nacht bei RT schwenken
Waschen
   10 Sekunden mit 1 x PBS schwenken, RT, Puffer abgießen
   2 x 15 Min. mit 1x PBS schwenken, RT, Puffer abgießen
Hybridisierung 2. Antikörper
   Inkubation der Membran in 25 ml Prehybridisierungspuffer + 25 µl (1:1000) Goat Anti-Rabbit IgG Horseradish Peroxidase Conjugate (HRP) (Southern Biotech, über Biozol Cat. No.: 4050 - 05) 60 Min. bei RT schwenken
Waschen
   10 Sekunden mit 1 x PBS schwenken, RT, Puffer abgießen
   2 x 15 Min. mit 1 x PBS schwenken, RT, Puffer abgießen
Detektion über Chemilumineszenz
   Lumi-Light Western Blotting Substrate (Roche, Cat. No.: 2015200) vorbereiten: Lumi-Light Luminol / Enhancer Solution und Lumi-Light Stable Peroxide Solution im Verhältnis 1:1 mischen: 3 ml / NC-Membran.
   Blot 5 Min. bei RT mit Lumi-Light Western Blotting Substrate inkubieren, Überschuss ablaufen lassen, Membran mit Frischhaltefolie bedecken und sofort einen X-Ray Film (Kodak, X-OMAT) auflegen, 2 Min. exponieren, entwickeln und fixieren. Bei schwachen Signalen wird die Exposition über einen längeren Zeitraum wiederholt.
Puffer
   Prehybridisierungspuffer: 5 % Magermilchpulver in 1x PBS
   10x PBS: 100 mM NaH₂PO₄, 1,5 M NaCl, pH 7,5 mit NaOH, 0,5 % Triton 100
   1x PBS: 10x PBS mit vollentsalztem Wasser 1:10 verdünnen
Quantifizierung
   Eine quantitative Auswertung erfolgte nach Einscannen des Immunoblots mit Biorad GS-800 Calibrated Densitometer mittels der Quantity One 1-D-Analysis Software (Biorad) durch Vergleich mit dem aufgetragenen Standard.

In Tabelle 3 sind die auf diese Weise bestimmten Interferonα2b-Ausbeuten im Fermentationsüberstand dargestellt.

**Tabelle 3: Interferonα2b-Ausbeuten im Fermentationsüberstand nach 72 h Fermentation**

| Stamm | Interferonα2b (mg/l) |
|---|---|
| W3110Δlpp/pIFN | 530 |
| W3110lpp1/pIFN | 510 |
| W3110lpp3/pIFN | 570 |

### Beispiel 7: Fermentative Produktion von Fab-Antikörperfragmenten mit lpp-Mutanten im 10 l-Maßstab

Mit Hilfe einer *lpp*-Mutante von *E. coli* können auch funktionelle Fab-Antikörperfragmente extrazellulär hergestellt werden. Dabei muss die Zelle die entsprechenden Fragmente der leichten Kette, welche die Domänen V_{L} und C_{L} umfasst, und der schweren Kette, welche die Domänen V_{H} und CH1 umfasst, gleichzeitig synthetisieren und dann in das Periplasma und schließlich in das Fermentationsmedium sekretieren. Außerhalb des Cytoplasmas erfolgt dann die Assemblierung der beiden Ketten zum funktionellen Fab-Fragment.

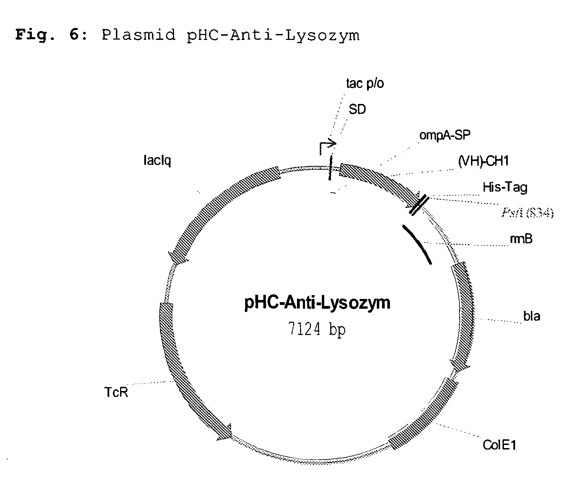

Das vorliegende Beispiel beschreibt die Produktion eines Fab-Fragments des gut charakterisierten Anti-Lysozym-Antikörpers D1.3.
Als Ausgangsvektor für die Klonierung und Expression der Gene des Anti-Lysozym-Fab-Fragments diente das Plasmid pJF118ut. In dieses Plasmid wurden in zwei aufeinanderfolgenden Schritten die beiden Leserahmen für die schwere Kette (V_{H}-C_{H}1-Domänen) bzw. für die leichte Kette (V_{L}-C_{L}-Domänen) des Anti-Lysozym-Fab-Fragments jeweils inklusive einer Signalsequenz kloniert. Dazu wurde wie folgt vorgegangen:
Das DNS-Fragment mit der SEQ ID No. 12 (schwere Kette) wurde mittels Gensynthese hergestellt und umfasst eine Genfusion bestehend aus der Signalsequenz des ompA-Gens von *E. coli* und dem Leserahmen für die schwere Kette (V_{H}-CH1) des Fab-Fragments. An diesen Leserahmen schließen direkt sechs Histidin-Codons an und bilden somit den C-Terminus des Fusionsproteins. Über diesen His-Tag ist später eine einfache Aufreinigung des vollständig assemblierten Fab-Fragments mittels Affinitätschromatographie möglich. Dieses DNS-Fragment wurde mit den Restriktionsenzymen EcoRI und PstI geschnitten und mit dem Expressionsvektor pJF118ut, der mit denselben Restriktionsenzymen geschnitten worden war, ligiert. Das aus dieser Klonierung resultierende Plasmid, bei dem die Expression des Gens für die schwere Kette unter der Kontrolle des tac-Promotors liegt, wurde mit pHC-Anti-Lysozym bezeichnet (Fig. 6).

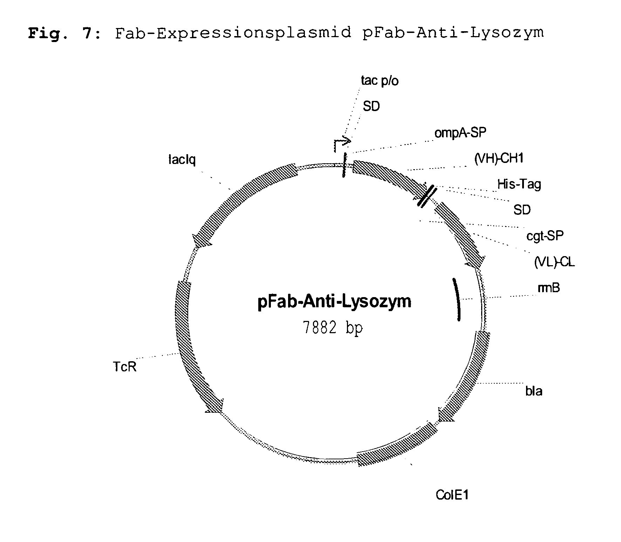

Das DNS-Fragment mit der SEQ ID No. 13 (leichte Kette) wurde ebenfalls mittels Gensynthese hergestellt und umfasst eine Genfusion bestehend aus der Signalsequenz einer CGTase (SEQ ID No. 3) und dem Leserahmen für die leichte Kette (V_{L}-C_{L}) des Fab-Fragments. Dieses DNS-Fragment wurde zunächst mit dem Restriktionsenzym PstI geschnitten und anschließend mit dem Vektor pHC-Anti-Lysozym, der mit demselben Restriktionsenzym geschnittenen worden war, ligiert. Das daraus resultierende Plasmid wurde mit pFab-Anti-Lysozym (Fig. 7) bezeichnet. Auf diese Weise wurde ein artifizielles Operon bestehend aus den jeweiligen Leserahmen für die schwere und die leichte Kette erzeugt, welches unter der Kontrolle des tac-Promotors steht. Damit ist durch Zugabe eines Induktors (z. B. IPTG) eine synchrone Expression der beiden Gene möglich.

Für die Herstellung des Anti-Lysozym-Fab-Fragments wurden die Stämme W3110Δlpp, W3110lpp1 und W3110lpp3 jeweils mit dem Plasmid pFab-Anti-Lysozym mit der CaCl₂-Methode transformiert. Die Selektion auf plasmidhaltige Zellen erfolgte mittels Ampicillin (100 mg/l).

Die Produktion des Anti-Lysozym-Fab-Fragments im 10 l-Maßstab erfolgte analog dem in Beispiel 4 beschriebenen Verfahren mit den Stämmen W3110Δlpp/pFab-Anti-Lysozym, W3110lpp1/pFab-Anti-Lysozym und W3110lpp3/pFab-Anti-Lysozym. Nach 72 h Fermentation wurden Proben entnommen und anschließend die Zellen durch Zentrifugation vom Fermentationsmedium abgetrennt.

Die Reinigung des Anti-Lysozym-Fab-Fragments aus den Fermentationsüberständen erfolgte mittels Affinitätschromatographie, wie in Skerra (1994, Gene 141, 79 - 84) beschrieben.

Die Quantifizierung sowie die Bestimmung der Aktivität des gereinigten Anti-Lysozym-Fab-Fragments erfolgte über einen ELISA-Test mit Lysozym als Antigen (Skerra, 1994, Gene 141, 79 - 84).

In Tabelle 4 sind die Ausbeuten an funktionellem Anti-Lysozym-Fab-Fragment aufgelistet, die aus jeweils 20 ml Fermentationsüberstand nach 72 h Fermentation isoliert werden konnten.

**Tabelle 4: Anti-Lysozym-Fab-Fragment-Ausbeuten im Fermentationsüberstand nach 72 h Fermentation**

| Stamm | aus 20 ml Überstand gereinigtes Anti-Lysozym-Fab-Fragment [mg] | Anti-Lysozym-Fab- Fragment-Ausbeute [g/l] im Fermentationsüberstand (hochgerechnet) |
|---|---|---|
| W3110Δlpp/pFab-Anti-Lysozym | 27 | 1,3 |
| W3110lpp1/pFab-Anti-Lysozym | 20 | 1,0 |
| W3110lpp3/pFab-Anti-Lysozym | 30 | 1,5 |

### Beispiel 8: Fermentative Produktion von Volllängen-Antikörpern mit lpp-Mutanten im 10 l-Maßstab

Mit Hilfe einer *lpp*-Mutante von *E. coli* können auch funktionelle Volllängen-Antikörper extrazellulär hergestellt werden. Analog zur Herstellung der Fab-Fragmente muss die Zelle die leichte und die schwere Kette des Antikörpers gleichzeitig synthetisieren und dann in das Periplasma und schließlich in das Fermentationsmedium sekretieren. Außerhalb des Cytoplasmas erfolgt dann die Assemblierung der beiden Ketten zum funktionellen Volllängen-Antikörper.

Das vorliegende Beispiel beschreibt die Produktion des Anti-Tissue factor (αTF) IgG1-Antikörpers.

Als Ausgangsvektor für die Klonierung und Expression der Gene des Anti-αTF-Antikörpers diente das Plasmid pJF118ut. In dieses Plasmid wurden in zwei aufeinanderfolgenden Schritten die beiden Leserahmen für die schwere Kette bzw. für die leichte Kette des Anti-αTF-Antikörpers jeweils inklusive einer Signalsequenz kloniert.

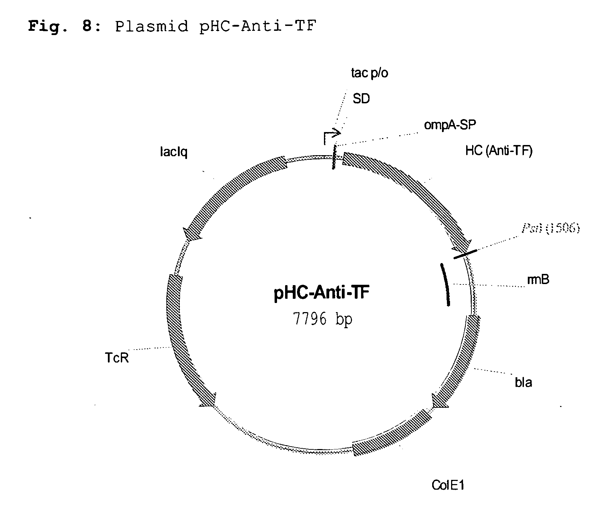

Dazu wurde wie folgt vorgegangen:
Das DNS-Fragment mit der SEQ ID No. 14 (schwere Kette) wurde mittels Gensynthese hergestellt und umfasst eine Genfusion bestehend aus der Signalsequenz des ompA-Gens von *E. coli* und dem Leserahmen für die schwere Kette des Anti-αTF-Antikörpers. Dieses DNS-Fragment wurde zunächst mit den Restriktionsenzymen EcoRI und PstI geschnitten und mit dem Expressionsvektor pJF118ut, der mit denselben Restriktionsenzymen geschnitten worden war, ligiert. Das aus dieser Klonierung resultierende Plasmid, bei dem die Expression des Gens für die schwere Kette unter der Kontrolle des tac-Promotors liegt, wurde mit pHC-Anti-TF bezeichnet (Fig. 8)

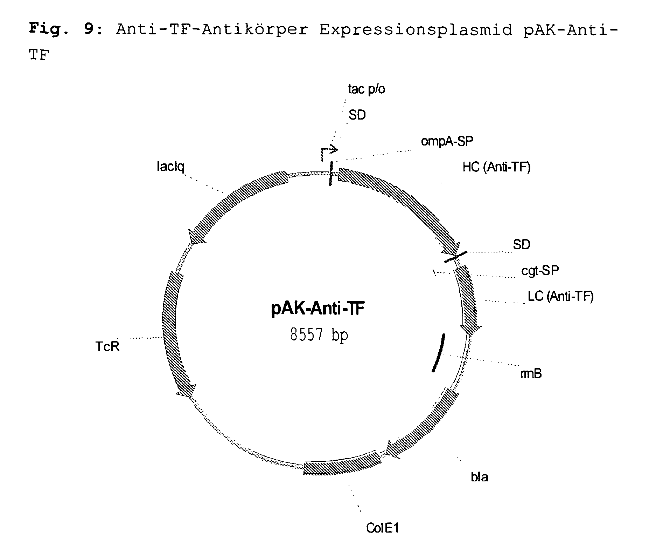

Das DNS-Fragment mit der SEQ ID No. 15 (leichte Kette) wurde ebenfalls mittels Gensynthese hergestellt und umfasst eine Genfusion bestehend aus der Signalsequenz einer CGTase (SEQ ID No. 3) und dem Leserahmen für die leichte Kette des Anti-αTF-Antikörpers. Dieses DNS-Fragment wurde zunächst mit dem Restriktionsenzym PstI geschnitten und anschließend mit dem Vektor pHC-Anti-TF, der mit demselben Restriktionsenzym geschnittenen worden war, ligiert. Das daraus resultierende Plasmid wurde mit pAK-Anti-TF (Fig. 9) bezeichnet. Auf diese Weise wurde ein artifizielles Operon bestehend aus den jeweiligen Leserahmen für die schwere und die leichte Kette erzeugt, welches unter der Kontrolle des tac-Promotors steht. Damit ist durch Zugabe eines Induktors (z. B. IPTG) eine synchrone Expression der beiden Gene möglich.

Für die Herstellung des Anti-αTF-Antikörpers wurden die Stämme W3110Δlpp, W3110lpp1 und W3110lpp3 jeweils mit dem Plasmid pAK-Anti-TF mit der CaCl₂-Methode transformiert. Die Selektion auf plasmidhaltige Zellen erfolgte mittels Ampicillin (100 mg/l).

Die Produktion des Anti-αTF-Antikörpers im 10 1-Maßstab erfolgte analog dem in Beispiel 4 beschriebenen Verfahren mit den Stämmen W3110Δlpp/pAK-Anti-TF, W3110lpp1/pAK-Anti-TF und W3110lpp3/pAK-Anti-TF. Nach 72 h Fermentation wurden Proben entnommen und anschließend die Zellen durch Zentrifugation vom Fermentationsmedium abgetrennt.

Die Quantifizierung des in das Fermentationsmedium sekretierten Anti-αTF-Antikörpers erfolgte über eine Aktivitäts-Bestimmung mit einem ELISA-Test mit löslichem Tissue factor als Antigen (Coating) und einem Peroxidase-konjugierten goat Anti-human F(ab')₂-Fragment als sekundärem Antikörper wie in Simmons et al. (2002, J. Immunol. Methods 263, 133 - 47) beschrieben.

In Tabelle 5 sind die auf diese Weise bestimmten Ausbeuten an funktionellem Anti-αTF-Antikörper aufgelistet.

**Tabelle 5: Anti-αTF-Antikörper-Ausbeuten im Fermentationsüberstand nach 72 h Fermentation**

| Stamm | Anti-αTF-Antikörper [mg/l] |
|---|---|
| W3110Δlpp/pAK-Anti-TF | 580 |
| W3110lpp1/pAK-Anti-TF | 600 |
| W3110lpp3/pAK-Anti-TF | 650 |

## Patentansprüche

1. Verfahren zur Produktion eines heterologen Proteins mittels eines E. coli-Stammes, welcher eine Mutation im lpp-Gen oder im Promotorbereich des lpp-Gens aufweist und ein Gen kodierend für ein heterologes Protein, welches mit einer Signalsequenz kodierend für ein Signalpeptid funktionell verknüpft ist, enthält, in einem Fermenter mit einem Volumen von mehr als 5 l, **dadurch gekennzeichnet, dass** die Mutation im lpp-Gen eine Substitution, eine Deletion oder eine Insertion eines oder mehrerer Nukleotide im lpp-Gen oder im Promotorbereich des lpp-Gens ist, welche dazu führt, dass das lpp-Gen nicht mehr oder nur noch vermindert exprimiert wird, so dass in den Zellen nur noch maximal 80 % der Lpp-Proteinmenge im Vergleich zu Zellen des Wildtyp-Stammes W3110 (ATTC: 27325) nachgewiesen werden kann oder die zu einer veränderten Aminosäuresequenz des Lpp-Proteines führt, welche mit einer Verminderung der Funktionalität des Lpp-Proteins einhergeht, worunter zu verstehen ist, dass die Resistenz der Zellen gegenüber Globomycin um mindestens den Faktor 2 im Vergleich zu dem lpp-Wildtypstamm W3110 erhöht ist, oder dass die Mutation im lpp-Gen, einen Austausch des Arginin-Rests an Position 77 von SEQ ID No. 2 gegen einen Cystein-Rest bewirkt oder einen Austausch des Glycin-Rests an Position 14 von SEQ ID No. 2 gegen einen Asparaginsäure-Rest bewirkt und in einem Fermentationsmedium enthaltend Ca²⁺-Ionen in einer Konzentration von 10 mg/l bis 5000 mg/l und Mg²⁺-Ionen in einer Konzentration größer 48 mg/l bis 5000 mg/l fermentiert wird, wobei der E. coli-Stamm das heterologe Protein in das Fermentationsmedium ausscheidet und das Protein vom Fermentationsmedium abgetrennt wird.

2. Verfahren nach Anspruch 1, **dadurch gekennzeichnet, dass** das heterologe Protein ein eukaryontisches Protein ist.

3. Verfahren nach einem der Ansprüche 1 oder 2, **dadurch gekennzeichnet, dass** das Protein eine oder mehrere Disulfid-Brücken enthält oder in funktioneller Form als Di- oder Multimer vorliegt.

4. Verfahren nach Anspruch 2, **dadurch gekennzeichnet, dass** das eukaryontische Protein ein Antikörper oder Antikörperfragment, ein Cytokin, ein Wachstumsfaktor, eine Proteinkinase oder ein Proteinhormon ist.

5. Verfahren nach Anspruch 2, 3 oder 4, **dadurch gekennzeichnet, dass** ein Antikörperfragment in technischem Maßstab in einer extrazellulären Ausbeute von mehr als 1 g/l hergestellt wird.

6. Verfahren nach einem der Ansprüche 1 bis 5, **dadurch gekennzeichnet, dass** das Gen kodierend für ein Signalpeptid ausgewählt ist aus der Gruppe der Gene kodierend für die Signalsequenz des phoA- oder ompA-Gens von E. coli, oder die Signalsequenz mit SEQ ID No. 3.

7. Verfahren nach einem der Ansprüche 3 bis 6 für die Sekretion eines Proteins, das aus mehreren unterschiedlichen Untereinheiten besteht, **dadurch gekennzeichnet, dass** das 5'-Ende des Gens der zu produzierenden Untereinheit des Proteins in frame mit dem 3'-Ende einer Signalsequenz für den Protein-Export verknüpft ist, wobei die Gene der verschiedenen Untereinheiten des Proteins mit unterschiedlichen Signalsequenzen verknüpft sind.

8. Verfahren nach einem der Ansprüche 1 bis 7, **dadurch gekennzeichnet, dass** die Fermentation in einem Fermenter mit einem Volumen von mehr als 50 1 erfolgt.

9. Verfahren nach einem der Ansprüche 1 bis 8, **dadurch gekennzeichnet, dass** ein Fermentationsmedium, welches Ca²⁺-Ionen in einer Konzentration 40 mg/l bis 5000 mg/l, und Mg²⁺-Ionen in einer Konzentration größer 48 mg/l bis maximal 5000 mg/l enthält, eingesetzt wird.

10. Verfahren nach einem der Ansprüche 1 bis 9, **dadurch gekennzeichnet, dass** es sich um ein Minimalsalzmedium handelt.

11. Verfahren nach einem der Ansprüche 1 bis 10, **dadurch gekennzeichnet, dass** die Fermentation über einen Zeitraum von 24 - 72 h erfolgt.

## Claims

1. Process for producing a heterologous protein by means of an E. coli strain which has a mutation in the lpp gene or in the promoter region of the lpp gene, and contains a gene coding for a heterologous protein which is functionally linked to a signal sequence coding for a signal peptide, in a fermenter with a volume of more than 5 1, **characterized in that** the mutation in the lpp gene is a substitution, a deletion or an insertion of one or more nucleotides in the lpp gene or in the promoter region of the lpp gene, leading to the lpp gene no longer being expressed or being expressed to only a reduced extent, so that only a maximum of 80% of the amount of Lpp protein is detectable in the cells by comparison with cells of the wild-type strain W3110 (ATTC: 27325), or leading to an altered amino acid sequence of the Lpp protein which is associated with a reduction in the functionality of the Lpp protein, by which is meant that the resistance of the cells to globomycin is increased by a factor of at least 2 by comparison with the lpp wild-type strain W3110, or that the mutation in the lpp gene brings about a replacement of the arginine residue at position 77 of SEQ ID No. 2 by a cysteine residue or brings about a replacement of the glycine residue at position 14 of SEQ ID No. 2 by an aspartic acid residue, and fermentation is carried out in a fermentation medium comprising Ca²⁺ ions in a concentration of 10 mg/l to 5000 mg/l and Mg²⁺ ions in a concentration above 48 mg/l to 5000 mg/l, with the E. coli strain secreting the heterologous protein into the fermentation medium, and the protein being removed from the fermentation medium.

2. Process according to Claim 1, **characterized in that** the heterologous protein is a eukaryotic protein.

3. Process according to Claim 1 or 2, **characterized in that** the protein comprises one or more disulfide bridges or is in the form of a dimer or multimer in functional form.

4. Process according to Claim 2, **characterized in that** the eukaryotic protein is an antibody or antibody fragment, a cytokine, a growth factor, a protein kinase or a protein hormone.

5. Process according to Claim 2, 3 or 4, **characterized in that** an antibody fragment is produced on the industrial scale in an extracellular yield of more than 1 g/l.

6. Process according to any of Claims 1 to 5, **characterized in that** the gene coding for a signal peptide is selected from the group of genes coding for the signal sequence of the phoA or ompA gene of E. coli, or the signal sequence having SEQ ID No. 3.

7. Process according to any of Claims 3 to 6 for secreting a protein which consists of a plurality of different subunits, **characterized in that** the 5' end of the gene of the protein subunit to be produced is linked in frame to the 3' end of a signal sequence for protein export, with the genes of the different subunits of the protein being linked to different signal sequences.

8. Process according to any of Claims 1 to 7, **characterized in that** the fermentation takes place in a fermenter with a volume of more than 50 1.

9. Process according to any of Claims 1 to 8, **characterized in that** a fermentation medium which comprises Ca²⁺ ions in a concentration of 40 mg/l to 5000 mg/l, and Mg²⁺ ions in a concentration of more than 48 mg/l up to a maximum of 5000 mg/l, is employed.

10. Process according to any of Claims 1 to 9, **characterized in that** a minimal salt medium is involved.

11. Process according to any of Claims 1 to 10, **characterized in that** the fermentation takes place over a period of 24 - 72 h.

## Revendications

1. Procédé pour la production d'une protéine hétérologue au moyen d'une souche d'E. coli qui présente une mutation dans le gène lpp ou dans la zone du promoteur du gène lpp et qui contient un gène codant pour une protéine hétérologue, qui est lié de manière fonctionnelle à une séquence signal codant pour un peptide signal, dans un fermenteur présentant un volume supérieur à 5 1, **caractérisé en ce que** la mutation dans le gène lpp est une substitution, une délétion ou une insertion d'un ou de plusieurs nucléotides dans le gène lpp ou dans la zone du promoteur du gène lpp, qui fait que le gène lpp n'est plus exprimé ou seulement de manière réduite, de manière telle qu'on ne peut plus détecter dans les cellules qu'au maximum 80% de la quantité de protéine Lpp par rapport aux cellules de la souche de type sauvage W3110 (ATTC : 27325) ou qui conduit à une séquence d'acides aminés modifiée de la protéine Lpp, qui va de paire avec une diminution de la fonctionnalité de la protéine Lpp, ce qui signifie que la résistance des cellules à la globomycine est augmentée au moins d'un facteur 2 par rapport à la souche lpp de type sauvage W3110 ou **en ce que** la mutation dans le gène lpp provoque un remplacement du résidu arginine en position 77 de la séquence SEQ ID No. 2 par un résidu cystéine ou un remplacement du résidu glycine en position 14 de la séquence SEQ ID No. 2 par un résidu acide aspartique et on fermente dans un milieu de fermentation contenant des ions Ca²⁺ en une concentration de 10 mg/l à 5000 mg/l et des ions Mg²⁺ en une concentration supérieure à 48 mg/l jusqu'à 5000 mg/l, la souche d'E. coli excrétant la protéine hétérologue dans le milieu de fermentation et la protéine étant séparée du milieu de fermentation.

2. Procédé selon la revendication 1, **caractérisé en ce que** la protéine hétérologue est une protéine eucaryote.

3. Procédé selon l'une quelconque des revendications 1 ou 2, **caractérisé en ce que** la protéine contient un ou plusieurs ponts disulfure ou se trouve sous forme fonctionnelle sous forme de dimère ou de multimère.

4. Procédé selon la revendication 2, **caractérisé en ce que** la protéine eucaryote est un anticorps ou un fragment d'anticorps, une cytokine, un facteur de croissance, une protéine-kinase ou une hormone protéinique.

5. Procédé selon la revendication 2, 3 ou 4, **caractérisé en ce qu'**on produit un fragment d'anticorps à l'échelle industrielle avec un rendement extracellulaire de plus de 1 g/l.

6. Procédé selon l'une quelconque des revendications 1 à 5, **caractérisé en ce que** le gène codant pour un peptide signal est choisi dans le groupe des gènes codant pour la séquence signal du gène phoA ou ompA d'E. coli, ou la séquence de signal de séquence SEQ ID No. 3.

7. Procédé selon l'une quelconque des revendications 3 à 6 pour la sécrétion d'une protéine qui est constituée par plusieurs sous-unités différentes, **caractérisé en ce que** l'extrémité 5' du gène de la sous-unité à produire de la protéine est liée dans le cadre à l'extrémité 3' d'une séquence signal pour l'export de la protéine, les gènes des différentes sous-unités de la protéine étant liés à différentes séquences signal.

8. Procédé selon l'une quelconque des revendications 1 à 7, **caractérisé en ce que** la fermentation est réalisée dans un fermenteur présentant un volume supérieur à 50 1.

9. Procédé selon l'une quelconque des revendications 1 à 8, **caractérisé en ce qu'**on utilise un milieu de fermentation qui contient des ions Ca²⁺ en une concentration de 40 mg/l à 5000 mg/l, et des ions Mg²⁺ en une concentration supérieure à 48 mg/l jusqu'à maximum 5000 mg/l.

10. Procédé selon l'une quelconque des revendications 1 à 9, **caractérisé en ce qu'**il s'agit d'un milieu à teneur minimale en sel.

11. Procédé selon l'une quelconque des revendications 1 à 10, **caractérisé en ce que** la fermentation est réalisée sur un laps de temps de 24-72 h.


### SEQUENZPROTOKOLL

<110> Consortium fuer elektrochemische Industrie GmbH
<120> Verfahren zur fermentativen Herstellung von Proteinen
<130> Co10615
<140>
   <141>
<160> 15
<170> PatentIn Vers. 2.0
<210> 1
   <211> 237
   <212> DNA
   <213> Escherichia coli
<220>
   <221> gene
   <222> (1) .. (237)
   <223> lpp-Wildtypgen
<400> 1
   atgaaagcta ctaaactggt actgggcgcg gtaatcctgg gttctactct gctggcaggt 60
   tgctccagca acgctaaaat cgatcagctg tcttctgacg ttcagactct gaacgctaaa 120
   gttgaccagc tgagcaacga cgtgaacgca atgcgttccg acgttcaggc tgctaaagat 180
   gacgcagctc gtgctaacca gcgtctggac aacatggcta ctaaataccg caagtaa    237
<210> 2
   <211> 78
   <212> PRT
   <213> Escherichia coli
<220>
   <221> SIGNAL
   <222> (1) .. (20)
   <223> Lpp-Signalpeptid
<220>
   <221> PEPTIDE
   <222> (21)..(78)
   <223> Aminosäure-Sequenz des prozessierten Lpp-Proteins
<400> 2
   Met Lys Ala Thr Lys Leu Val Leu Gly Ala Val Ile Leu Gly Ser Thr
   1 5 10 15
   Leu Leu Ala Gly Cys Ser Ser Asn Ala Lys Ile Asp Gln Leu Ser Ser
   20 25 30
   Asp Val Gln Thr Leu Asn Ala Lys Val Asp Gln Leu Ser Asn Asp Val
   35 40 45
   Asn Ala Met Arg Ser Asp Val Gln Ala Ala Lys Asp Asp Ala Ala Arg
   50 55 60
   Ala Asn Gln Arg Leu Asp Asn Met Ala Thr Lys Tyr Arg Lys
   65 70 75
<210> 3
   <211> 90
   <212> DNA
   <213> Klebsiella pneumoniae
<220>
   <223> CGTase-Signalsequenz
<400> 3
   atgaaaagaa accgtttttt taatacctcg gctgctattg ccatttcgat tgcattaaat 60
   actttttttt gtagcatgca gacgattgct 90
<210> 4
   <211> 70
   <212> DNA
   <213> Artificial Sequence
<220>
   <223> Oligonukleotid lpp1
<400> 4
   cagcgttcga tgcttctttg agcgaacgat caaaaataag tgccttccca catatgaata 60
   tcctccttag 70
<210> 5
   <211> 70
   <212> DNA
   <213> Artificial Sequence
<220>
   <223> Oligonukleotid lpp2
<400> 5
   gcgcacaatg tgcgccattt ttcacttcac aggtactatt acttgcggta gtgtaggctg 60
   gagctgcttc 70
<210> 6
   <211> 23
   <212> DNA
   <213> Artificial Sequence
<220>
   <223> Oligonukleotid pykF
<400> 6
   ctcgcgcagt acgtaaatac ttc 23
<210> 7
   <211> 21
   <212> DNA
   <213> Artificial Sequence
<220>
   <223> Oligonukleotid ynhG2
<400> 7
   cgtctttgct tacactcacg c 21
<210> 8
   <211> 452
   <212> DNA
   <213> Artificial Sequence
<220>
   <223> DNS-Molekuel, welches das lpp1-Allel enthaelt
<220>
   <221> allele
   <222> (9) .. (245)
   <223> Leserahmen des lpp1-Allels
<220>
   <221> mutation
   <222> (236)
   <223> Basensubstitution (C zu T) im lpp1-Allel
<400> 8
   cgggatccat gaaagctact aaactggtac tgggcgcggt aatcctgggt tctactctgc 60
   tggcaggttg ctccagcaac gctaaaatcg atcagctgtc ttctgacgtt cagactctga 120
   acgctaaagt tgaccagctg agcaacgacg tgaacgcaat gcgttccgac gttcaggctg 180
   ctaaagatga cgcagctcgt gctaaccagc gtctggacaa catggctact aaatactgca 240
   agtaatagta cctgtgaagt gaaaaatggc gcacattgtg cgccattttt tttgtctgcc 300
   gtttaccgct actgcgtcac gcgtaacata ttcccttgct ctggttcacc attctgcgct 360
   gactctactg aaggcgcatt gctggctgcg ggagttgctc cactgctcac cgaaaccgga 420
   taccctgccc gacgatacaa cgctggatcc cg                               452
<210> 9
   <211> 455
   <212> DNA
   <213> Artificial Sequence
<220>
   <223> DNS-Molekuel, welches das lpp3-Allel enthaelt
<220>
   <221> mutation
   <222> (251)
   <223> Basensubstitution (G zu A) im lpp3-Allel
<400> 9
   cgggatcctc tcactttatc taagatgaat ccgatggaag catcctgttt tctctcaatt 60
   tttttatcta aaacccagcg ttcgatgctt ctttgagcga acgatcaaaa ataagtgcct 120
   tcccatcaaa aaaatattct caacataaaa aactttgtgt aatacttgta acgctacatg 180
   gagattaact caatctagag ggtattaata atgaaagcta ctaaactggt actgggcgcg 240
   gtaatcctgg attctactct gctggcaggt tgctccagca acgctaaaat cgatcagctg 300
   tcttctgacg ttcagactct gaacgctaaa gttgaccagc tgagcaacga cgtgaacgca 360
   atgcgttccg acgttcaggc tgctaaagat gacgcagctc gtgctaacca gcgtctggac 420
   aacatggcta ctaaataccg caagtaagga tcccg                            455
<210> 10
   <211> 2065
   <212> DNA
   <213> Klebsiella pneumoniae
<220>
   <223> DNS-Molekuel, welches das CGTase-Gen enthaelt
<220>
   <221> sig_peptide
   <222> (9) .. (93)
   <223> Signalsequenz des CGTase-Gens
<220>
   <221> allele
   <222> (99)..(1971)
   <223> CGTase-Strukturgen
<400> 10
   ataatgaaaa gaaaccgttt ttttaatacc tcggctgcta ttgccatttc gattgcatta 60
   aatacttttt tttgtagcat gcagacgatt gctgctgaac cagaagaaac ttatcttgat 120
   tttcgtaagg agacgatata ttttctattc cttgatcgtt tcagcgatgg agatccaagt 180
   aataatgcag ggtttaattc tgcaacctac gatcctaata atttaaaaaa atatactgga 240
   ggagatctcc gggggttgat taataaacta ccctatttaa aatcacttgg tgttacttca 300
   atctggatta ctcccccaat cgataatgtg aataatactg atgctgctgg caatactgga 360
   tatcatggtt attggggaag agattatttt cgtatagatg aacattttgg caatctcgat 420
   gatttcaaag aactgactag tttgatgcat agtcctgatt ataatatgaa actggttctt 480
   gattatgccc ctaatcattc gaatgctaat gatgaaaatg aatttggtgc actatatcgt 540
   gatggtgtgt ttattactga ttatcctacg aatgttgccg ccaatacggg ctggtatcat 600
   cacaatggtg gggtaacgaa ctggaatgat ttcttccaag tgaagaatca taatctattc 660
   aatctatcag acctcaatca atccaatact gatgtctacc agtacttgtt ggatggttct 720
   aaattttgga tcgatgctgg tgtggatgct atcaggattg atgccatcaa gcatatggac 780
   aagtctttta tacagaaatg gaccagcgat atttatgatt acagtaagtc tatcggccgg 840
   gaaggatttt ttttcttcgg tgaatggttt ggtgccagtg cgaatactac aacaggtgtt 900
   gatggtaatg ctatcgatta cgccaacact tccgggtcag cgttgctgga ttttggattc 960
   cgcgatactt tagaaagagt tttggtagga cgtagcggaa atacaatgaa aacgttaaat 1020
   agttatctga taaaaagaca aacagtcttt accagtgatg actggcaggt tgtttttatg 1080
   gataaccatg atatggcacg cattggtacc gctctgcgtt caaacgccac tacttttggt 1140
   cctggaaata atgaaaccgg tggaagtcag agtgaagctt ttgctcagaa acgtatagac 1200
   ctcggtctgg ttgcgacaat gactgtacgt ggtattcctg ccatttatta tggtactgaa 1260
   cattatgccg ctaactttac ctctaacagt tttggtcaag ttggcagtga tccttacaac 1320
   cgagagaaaa tgccaggatt tgatacggaa agtgaggctt tctccattat taaaacactg 1380
   ggtgacctaa ggaaaagtag cccggcaatt caaaatggaa cttatactga actatgggtt 1440
   aatgatgata tattagtatt tgagcggcgt tctgggaacg atattgttat tgttgcactt 1500
   aatcgtggtg aggctaacac aattaatgtt aaaaatatag cggttcctaa tggggtatat 1560
   ccgagtttga ttgggaataa tagtgtttca gtagcaaata aacggacaac actaacactt 1620
   atgcaaaatg aagctgttgt cattcgctca caatcagatg atgcggagaa ccctacagta 1680
   caaagcataa acttcacatg taataacggt tatacgattt caggtcaaag tgtttatatt 1740
   attggtaata tacctcagtt aggtggttgg gacttaacta aagcggtaaa aatatcaccg 1800
   acacaatatc cacaatggag tgcgagctta gagcttcctt ctgacttaaa tgttgaatgg 1860
   aagtgtgtga aacgtaatga aaccaatccg acggctaatg ttgagtggca gtctggtgca 1920
   aataaccagt tcaatagcaa tgacacacaa acaacgaatg gctcgtttta attaaaattt 1980
   agtggaccag cgttccaatc gatggtccac tattcgtact ccggccataa ttatttttga 2040
   ctaatactct tacaaatttt caacc 2065
<210> 11
   <211> 652
   <212> DNA
   <213> Artificial Sequence
<220>
   <223> DNS-Molekuel, welches das Interferon alpha2b-Gen enthaelt
<220>
   <221> sig_peptide
   <222> (24)..(113)
   <223> CGTase-Signalpeptid
<220>
   <221> allele
   <222> (114)..(611)
   <223> Interferon alpha2b-Gen
<400> 11
   cagaattcta aggaggaaat tatatgaaaa gaaaccgttt ttttaatacc tcggctgcta 60
   ttgccatttc gattgcatta aatacttttt tttgtagcat gcagacgatt gcttgtgact 120
   tacctcagac ccattcactg ggctcacgcc gtacgctgat gctgttagca cagatgcgtc 180
   gcatttctct gtttagttgt ttgaaagacc gtcatgattt tgggttcccg caagaagagt 240
   ttggtaatca gtttcagaaa gccgaaacta ttccggttct gcacgaaatg attcaacaga 300
   tttttaacct gttttcgaca aaggatagct ctgccgcgtg ggatgaaacc ttactggata 360
   agttctacac cgaactgtac cagcaactga atgatctgga agcatgcgtt atccagggcg 420
   tgggtgtcac agaaactccg ctgatgaagg aggacagcat tctggcggtg cgcaaatatt 480
   tccagcgtat cacgctgtat ctgaaagaga aaaaatattc gccatgcgcg tgggaggtcg 540
   tgcgcgcgga gatcatgcgc agtttctctt tgagcaccaa cctgcaagaa tccttgcgtt 600
   ccaaagaata atagtctaga agcttggctg ttttggcgga tgagctgcag aa         652
<210> 12
   <211> 769
   <212> DNA
   <213> Artificial Sequence
<220>
   <223> DNS-Molekuel, welches das Gen der schweren Kette des Anti-Lysozym-Fab-Fragments enthaelt
<220>
   <221> sig_peptide
   <222> (24) .. (86)
   <223> ompA-Signalsequenz
<220>
   <221> allele
   <222> (87)..(758)
   <223> Gen der schweren Kette des Anti-Lysozym-Fab-Fragments inkl. His-Tag (738-755)
<400> 12
   cagaattcta aggaggaaat tatatgaaaa agacagctat cgcgattgca gtggcactgg 60
   ctggtttcgc taccgtagcg caggctgaag ttaaactgca agaatccggt ccgggtctgg 120
   ttgctccgtc ccagtccctg tccatcacct gcaccgtttc cggtttctcc ctgaccggtt 180
   acggtgttaa ctgggttcgt cagccgccgg gtaaaggtct ggaatggctg ggtatgatct 240
   ggggtgacgg taacaccgac tacaactccg ctctgaaatc ccgtctgtcc atctccaaag 300
   acaactccaa atcccaggtt ttcctgaaaa tgaactccct gcacaccgac gacaccgctc 360
   gttactactg cgctcgtgaa cgtgactacc gtctggacta ctggggtcag ggtaccaccg 420
   ttaccgtttc ctccgctaaa accaccccgc cgtccgttta cccgctggct ccgggttccg 480
   ctgctcagac caactctatg gttaccctgg gttgcctggt taaaggttac ttcccggaac 540
   cggttaccgt tacctggaac tccggttccc tgtcctccgg ttgccacacc ttcccggctg 600
   ttctgcaatc cgacctgtac accctgtcct cctccgttac cgttccgtcc tccacctggc 660
   cgtccgaaac cgttacctgc aacgttgctc acccggcttc ctccaccaaa gttgacaaaa 720
   aaatcgttcc gcgtgaccat caccaccatc accattaata actgcagaa             769
<210> 13
   <211> 768
   <212> DNA
   <213> Artificial Sequence
<220>
   <223> DNS-Molekuel, welches das Gen der leichten Kette des Anti-Lysozym-Fab-Fragments enthaelt
<220>
   <221> sig_peptide
   <222> (26)..(115)
   <223> CGTase-Signalsequenz
<220>
   <221> allele
   <222> (116)..(757)
   <223> Gen der leichten Kette des Anti-Lysozym-Fab-Fragments
<400> 13
   aactgcagta catggagaaa ataaaatgaa aagaaaccgt ttttttaata cctcggctgc 60
   tattgccatt tcgattgcat taaatacttt tttttgtagc atgcagacga ttgctgacat 120
   cgaactgacc cagtccccgg cttccctgtc cgcttccgtt ggtgaaaccg ttaccatcac 180
   ctgccgtgct tccggtaaca tccacaacta cctggcttgg taccagcaga aacagggtaa 240
   atccccgcag ctgctggttt actacaccac caccctggct gacggtgttc cgtcccgttt 300
   ctccggttcc ggttccggta cccagtactc cctgaaaatc aactccctgc aaccggaaga 360
   cttcggttcc tactactgcc agcacttctg gtccaccccg cgtaccttcg gtggtggtac 420
   caaactggaa ctgaaacgtg ctgacgctgc tccgaccgtt tccatcttcc cgccgtcctc 480
   cgaacagctg acctccggtg gtgcttccgt tgtttgcttc ctgaacaact tctacccgaa 540
   agacatcaac gttaaatgga aaatcgacgg ttccgaacgt cagaacggtg ttctgaactc 600
   ctggaccgac caggactcca aagactccac ctactccatg tcctccaccc tgaccctgac 660
   caaagacgaa tacgaacgtc acaactccta cacctgcgaa gctacccaca aaacctccac 720
   ctccccgatc gttaaatcct tcaaccgtaa cgaataatag ctgcagaa              768
<210> 14
   <211> 1441
   <212> DNA
   <213> Artificial Sequence
<220>
   <223> DNS-Molekuel, welches das Gen fuer die schwere Kette des Anti-TF-Antikoerpers enthaelt
<220>
   <221> sig_peptide
   <222> (24)..(86)
   <223> ompA-Signalsequenz
<220>
   <221> allele
   <222> (87)..(1430)
   <223> Gen der schweren Kette des Anti-TF-Antikoerpers
<400> 14
   cagaattcta aggaggaaat tatatgaaaa agacagctat cgcgattgca gtggcactgg 60
   ctggtttcgc taccgtagcg caggctgagg ttcagctggt ggagtctggc ggtggcctgg 120
   tgcagccagg gggctcactc cgtttgtcct gtgcagcttc tggcttcaat attaaggagt 180
   actacatgca ctgggtccgt caggccccgg gtaagggcct ggaatgggtt ggattgattg 240
   atccagagca aggcaacacg atctatgacc cgaagttcca ggaccgtgcc actataagcg 300
   ctgacaattc caaaaacaca gcatacctgc aaatgaacag cctgcgtgct gaggacactg 360
   ccgtctatta ttgtgctcga gacacggccg cttacttcga ctactggggt caaggaaccc 420
   tggtcaccgt ctcctcggcc tccaccaagg gcccatcggt cttccccctg gcaccctcct 480
   ccaagagcac ctctgggggc acagcggccc tgggctgcct ggtcaaggac tacttccccg 540
   aaccggtgac ggtgtcgtgg aactcaggcg ccctgaccag cggcgtgcac accttcccgg 600
   ctgtcctaca gtcctcagga ctctactccc tcagcagcgt ggtgactgtg ccctctagca 660
   gcttgggcac ccagacctac atctgcaacg tgaatcacaa gcccagcaac accaaggtgg 720
   acaagaaagt tgagcccaaa tcttgtgaca aaactcacac atgcccaccg tgcccagcac 780
   ctgaactcct ggggggaccg tcagtcttcc tcttcccccc aaaacccaag gacaccctca 840
   tgatctcccg gacccctgag gtcacatgcg tggtggtgga cgtgagccac gaagaccctg 900
   aggtcaagtt caactggtac gtggacggcg tggaggtgca taatgccaag acaaagccgc 960
   gggaggagca gtacaacagc acgtaccgtg tggtcagcgt cctcaccgtc ctgcaccagg 1020
   actggctgaa tggcaaggag tacaagtgca aggtctccaa caaagccctc ccagccccca 1080
   tcgagaaaac catctccaaa gccaaagggc agccccgaga accacaggtg tacaccctgc 1140
   ccccatcccg ggaagagatg accaagaacc aggtcagcct gacctgcctg gtcaaaggct 1200
   tctatcccag cgacatcgcc gtggagtggg agagcaatgg gcagccggag aacaactaca 1260
   agaccacgcc tcccgtgctg gactccgacg gctccttctt cctctacagc aagctcaccg 1320
   tggacaagag caggtggcag caggggaacg tcttctcatg ctccgtgatg catgaggctc 1380
   tgcacaacca ctacacgcag aagagcctct ccctgtctcc gggtaaataa taactgcaga 1440
   a 1441
<210> 15
   <211> 771
   <212> DNA
   <213> Artificial Sequence
<220>
   <223> DNS-Molekuel, welches das Gen fuer die leichte Kette des Anti-TF-Antikoerpers enthaelt
<220>
   <221> sig_peptide
   <222> (26)..(115)
   <223> CGTase-Signalsequenz
<220>
   <221> allele
   <222> (116)..(760)
   <223> Gen der leichten Kette des Anti-TF-Antikoerpers
<400> 15
   aactgcagta catggagaaa ataaaatgaa aagaaaccgt ttttttaata cctcggctgc 60
   tattgccatt tcgattgcat taaatacttt tttttgtagc atgcagacga ttgctgatat 120
   ccagatgacc cagtccccga gctccctgtc cgcctctgtg ggcgataggg tcaccatcac 180
   gtgcagagcc agtcgcgaca tcaagagcta tctgaactgg tatcaacaga aaccaggaaa 240
   agctccgaaa gtactgattt actatgctac tagtctcgct gaaggagtcc cttctcgctt 300
   ctctggatcc ggttctggga cggattacac tctgaccatc agcagtctgc aaccagaaga 360
   cttcgcaact tattactgtc ttcagcacgg agagtctccg tggacatttg gacagggtac 420
   caaggtggag atcaaacgaa ctgtggctgc accatctgtc ttcatcttcc cgccatctga 480
   tgagcagttg aaatctggaa ctgcttctgt tgtgtgcctg ctgaataact tctatcccag 540
   agaggccaaa gtacagtgga aggtggataa cgccctccaa tcgggtaact cccaggagag 600
   tgtcacagag caggacagca aggacagcac ctacagcctc agcagcaccc tgacgctgag 660
   caaagcagac tacgagaaac acaaagtcta cgcctgcgaa gtcacccatc agggcctgag 720
   ctcgcccgtc acaaagagct tcaacagggg agagtgttaa tagctgcaga a          771